(19)

Europäisches
Patentamt

European
Patent Office

Office européen
des brevets

(11) **EP 3 812 770 A1**

(12) **EUROPEAN PATENT APPLICATION**
published in accordance with Art. 153(4) EPC

(43) Date of publication:
**28.04.2021 Bulletin 2021/17**

(21) Application number: **19787579.2**

(22) Date of filing: **02.04.2019**

(51) Int Cl.:
***G01N 33/68*** (2006.01)   ***G01N 1/04*** (2006.01)
***G01N 33/50*** (2006.01)

(86) International application number:
**PCT/JP2019/014667**

(87) International publication number:
**WO 2019/202972 (24.10.2019 Gazette 2019/43)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **19.04.2018 JP 2018080666**

(71) Applicants:
 • **The University of Tokyo**
  **Bunkyo-ku,**
  **Tokyo 113-8654 (JP)**
 • **Saraya Co., Ltd.**
  **Osaka-shi**
  **Osaka 546-0013 (JP)**

(72) Inventors:
 • **MINEMATSU Takeo**
  **Tokyo 113-8654 (JP)**
 • **SANADA Hiromi**
  **Tokyo 113-8654 (JP)**
 • **TAMAI Nao**
  **Tokyo 113-8654 (JP)**
 • **HIRATA Yoshihiko**
  **Kashiwara-shi, Osaka 582-0028 (JP)**
 • **ODA Yuka**
  **Kashiwara-shi, Osaka 582-0028 (JP)**

(74) Representative: **Regimbeau**
  **20, rue de Chazelles**
  **75847 Paris Cedex 17 (FR)**

(54) **METHOD AND KIT FOR ASSISTING DIAGNOSIS OF DISEASE IN SUBJECT**

(57)    A problem to be solved by the present invention is to provide a method of assisting diagnosis of the health condition of a subject by correcting variation in an amount of a collected disease marker, which is caused by variation in the skin barrier function of the subject, and acquiring information reflecting the health condition of the subject accurately, and to solve the problem, the present invention provides a method of assisting diagnosis of a disease in a subject using a disease marker and a reference marker, the method including: applying, to a skin surface of the subject, a sheet capable of generating an attractive force due to electrostatic interaction between the sheet and each of the disease and reference markers; detaching the sheet from the skin surface; measuring the amount of each of the disease and reference markers that are attached to the sheet; and acquiring information on the disease in the subject based on a value obtained by correcting the measured amount of the disease marker with the measured amount of the reference marker, wherein the reference marker is annexin A2.

EP 3 812 770 A1

SEQUENCE LISTING

<110>  The University of Tokyo, et al.

<120>  Method and kit for facilitating diagnosing a disease in a subject

<130>  226454PX

<160>  10

<170>  PatentIn version 3.5

<210>  1
<211>  1020
<212>  DNA
<213>  Homo sapiens

<220>
<221>  CDS
<222>  (1)..(1020)

<400>  1

```
atg tct act gtt cac gaa atc ctg tgc aag ctc agc ttg gag ggt gat        48
Met Ser Thr Val His Glu Ile Leu Cys Lys Leu Ser Leu Glu Gly Asp
1               5                   10                  15

cac tct aca ccc cca agt gca tat ggg tct gtc aaa gcc tat act aac        96
His Ser Thr Pro Pro Ser Ala Tyr Gly Ser Val Lys Ala Tyr Thr Asn
                20                  25                  30

ttt gat gct gag cgg gat gct ttg aac att gaa aca gcc atc aag acc       144
Phe Asp Ala Glu Arg Asp Ala Leu Asn Ile Glu Thr Ala Ile Lys Thr
            35                  40                  45

aaa ggt gtg gat gag gtc acc att gtc aac att ttg acc aac cgc agc       192
Lys Gly Val Asp Glu Val Thr Ile Val Asn Ile Leu Thr Asn Arg Ser
        50                  55                  60

aat gca cag aga cag gat att gcc ttc gcc tac cag aga agg acc aaa       240
Asn Ala Gln Arg Gln Asp Ile Ala Phe Ala Tyr Gln Arg Arg Thr Lys
65                  70                  75                  80

aag gaa ctt gca tca gca ctg aag tca gcc tta tct ggc cac ctg gag       288
Lys Glu Leu Ala Ser Ala Leu Lys Ser Ala Leu Ser Gly His Leu Glu
                85                  90                  95

acg gtg att ttg ggc cta ttg aag aca cct gct cag tat gac gct tct       336
Thr Val Ile Leu Gly Leu Leu Lys Thr Pro Ala Gln Tyr Asp Ala Ser
                100                 105                 110

gag cta aaa gct tcc atg aag ggg ctg gga acc gac gag gac tct ctc       384
Glu Leu Lys Ala Ser Met Lys Gly Leu Gly Thr Asp Glu Asp Ser Leu
            115                 120                 125

att gag atc atc tgc tcc aga acc aac cag gag ctg cag gaa att aac       432
Ile Glu Ile Ile Cys Ser Arg Thr Asn Gln Glu Leu Gln Glu Ile Asn
        130                 135                 140

aga gtc tac aag gaa atg tac aag act gat ctg gag aag gac att att       480
Arg Val Tyr Lys Glu Met Tyr Lys Thr Asp Leu Glu Lys Asp Ile Ile
145                 150                 155                 160
```

```
tcg gac aca tct ggt gac ttc cgc aag ctg atg gtt gcc ctg gca aag    528
Ser Asp Thr Ser Gly Asp Phe Arg Lys Leu Met Val Ala Leu Ala Lys
            165             170             175

ggt aga aga gca gag gat ggc tct gtc att gat tat gaa ctg att gac    576
Gly Arg Arg Ala Glu Asp Gly Ser Val Ile Asp Tyr Glu Leu Ile Asp
            180             185             190

caa gat gct cgg gat ctc tat gac gct gga gtg aag agg aaa gga act    624
Gln Asp Ala Arg Asp Leu Tyr Asp Ala Gly Val Lys Arg Lys Gly Thr
            195             200             205

gat gtt ccc aag tgg atc agc atc atg acc gag cgg agc gtg ccc cac    672
Asp Val Pro Lys Trp Ile Ser Ile Met Thr Glu Arg Ser Val Pro His
            210             215             220

ctc cag aaa gta ttt gat agg tac aag agt tac agc cct tat gac atg    720
Leu Gln Lys Val Phe Asp Arg Tyr Lys Ser Tyr Ser Pro Tyr Asp Met
225             230             235             240

ttg gaa agc atc agg aaa gag gtt aaa gga gac ctg gaa aat gct ttc    768
Leu Glu Ser Ile Arg Lys Glu Val Lys Gly Asp Leu Glu Asn Ala Phe
            245             250             255

ctg aac ctg gtt cag tgc att cag aac aag ccc ctg tat ttt gct gat    816
Leu Asn Leu Val Gln Cys Ile Gln Asn Lys Pro Leu Tyr Phe Ala Asp
            260             265             270

cgg ctg tat gac tcc atg aag ggc aag ggg acg cga gat aag gtc ctg    864
Arg Leu Tyr Asp Ser Met Lys Gly Lys Gly Thr Arg Asp Lys Val Leu
            275             280             285

atc aga atc atg gtc tcc cgc agt gaa gtg gac atg ttg aaa att agg    912
Ile Arg Ile Met Val Ser Arg Ser Glu Val Asp Met Leu Lys Ile Arg
            290             295             300

tct gaa ttc aag aga aag tac ggc aag tcc ctg tac tat tat atc cag    960
Ser Glu Phe Lys Arg Lys Tyr Gly Lys Ser Leu Tyr Tyr Tyr Ile Gln
305             310             315             320

caa gac act aag ggc gac tac cag aaa gcg ctg ctg tac ctg tgt ggt    1008
Gln Asp Thr Lys Gly Asp Tyr Gln Lys Ala Leu Leu Tyr Leu Cys Gly
            325             330             335

gga gat gac tga                                                     1020
Gly Asp Asp
```

```
<210>   2
<211>   339
<212>   PRT
<213>   Homo sapiens

<400>   2

Met Ser Thr Val His Glu Ile Leu Cys Lys Leu Ser Leu Glu Gly Asp
1               5               10              15


His Ser Thr Pro Pro Ser Ala Tyr Gly Ser Val Lys Ala Tyr Thr Asn
            20              25              30
```

Phe Asp Ala Glu Arg Asp Ala Leu Asn Ile Glu Thr Ala Ile Lys Thr
    35                      40                  45

Lys Gly Val Asp Glu Val Thr Ile Val Asn Ile Leu Thr Asn Arg Ser
    50                      55                  60

Asn Ala Gln Arg Gln Asp Ile Ala Phe Ala Tyr Gln Arg Arg Thr Lys
65                      70                  75                  80

Lys Glu Leu Ala Ser Ala Leu Lys Ser Ala Leu Ser Gly His Leu Glu
                85                  90                  95

Thr Val Ile Leu Gly Leu Leu Lys Thr Pro Ala Gln Tyr Asp Ala Ser
                100                 105                 110

Glu Leu Lys Ala Ser Met Lys Gly Leu Gly Thr Asp Glu Asp Ser Leu
        115                 120                 125

Ile Glu Ile Ile Cys Ser Arg Thr Asn Gln Glu Leu Gln Glu Ile Asn
    130                 135                 140

Arg Val Tyr Lys Glu Met Tyr Lys Thr Asp Leu Glu Lys Asp Ile Ile
145                 150                 155                 160

Ser Asp Thr Ser Gly Asp Phe Arg Lys Leu Met Val Ala Leu Ala Lys
                165                 170                 175

Gly Arg Arg Ala Glu Asp Gly Ser Val Ile Asp Tyr Glu Leu Ile Asp
                180                 185                 190

Gln Asp Ala Arg Asp Leu Tyr Asp Ala Gly Val Lys Arg Lys Gly Thr
        195                 200                 205

Asp Val Pro Lys Trp Ile Ser Ile Met Thr Glu Arg Ser Val Pro His
    210                 215                 220

Leu Gln Lys Val Phe Asp Arg Tyr Lys Ser Tyr Ser Pro Tyr Asp Met
225                 230                 235                 240

Leu Glu Ser Ile Arg Lys Glu Val Lys Gly Asp Leu Glu Asn Ala Phe
                245                 250                 255

Leu Asn Leu Val Gln Cys Ile Gln Asn Lys Pro Leu Tyr Phe Ala Asp
                260                 265                 270

Arg Leu Tyr Asp Ser Met Lys Gly Lys Gly Thr Arg Asp Lys Val Leu
        275                 280                 285

24

EP 3 812 770 A1

```
Ile Arg Ile Met Val Ser Arg Ser Glu Val Asp Met Leu Lys Ile Arg
    290             295             300


Ser Glu Phe Lys Arg Lys Tyr Gly Lys Ser Leu Tyr Tyr Tyr Ile Gln
305             310             315             320


Gln Asp Thr Lys Gly Asp Tyr Gln Lys Ala Leu Leu Tyr Leu Cys Gly
                325             330             335


Gly Asp Asp



<210>  3
<211>  1074
<212>  DNA
<213>  Homo sapiens


<220>
<221>  CDS
<222>  (1)..(1074)


<400>  3
atg ggc cgc cag cta gcg ggg tgt gga gac gct ggg aag aag gct tcc      48
Met Gly Arg Gln Leu Ala Gly Cys Gly Asp Ala Gly Lys Lys Ala Ser
1               5               10              15


ttc aaa atg tct act gtt cac gaa atc ctg tgc aag ctc agc ttg gag     96
Phe Lys Met Ser Thr Val His Glu Ile Leu Cys Lys Leu Ser Leu Glu
                20              25              30


ggt gat cac tct aca ccc cca agt gca tat ggg tct gtc aaa gcc tat    144
Gly Asp His Ser Thr Pro Pro Ser Ala Tyr Gly Ser Val Lys Ala Tyr
            35              40              45


act aac ttt gat gct gag cgg gat gct ttg aac att gaa aca gcc atc    192
Thr Asn Phe Asp Ala Glu Arg Asp Ala Leu Asn Ile Glu Thr Ala Ile
        50              55              60


aag acc aaa ggt gtg gat gag gtc acc att gtc aac att ttg acc aac    240
Lys Thr Lys Gly Val Asp Glu Val Thr Ile Val Asn Ile Leu Thr Asn
65              70              75              80


cgc agc aat gca cag aga cag gat att gcc ttc gcc tac cag aga agg    288
Arg Ser Asn Ala Gln Arg Gln Asp Ile Ala Phe Ala Tyr Gln Arg Arg
                85              90              95


acc aaa aag gaa ctt gca tca gca ctg aag tca gcc tta tct ggc cac    336
Thr Lys Lys Glu Leu Ala Ser Ala Leu Lys Ser Ala Leu Ser Gly His
                100             105             110


ctg gag acg gtg att ttg ggc cta ttg aag aca cct gct cag tat gac    384
Leu Glu Thr Val Ile Leu Gly Leu Leu Lys Thr Pro Ala Gln Tyr Asp
            115             120             125


gct tct gag cta aaa gct tcc atg aag ggg ctg gga acc gac gag gac    432
Ala Ser Glu Leu Lys Ala Ser Met Lys Gly Leu Gly Thr Asp Glu Asp
```

25

```
                130                      135                         140

     tct ctc att gag atc atc tgc tcc aga acc aac cag gag ctg cag gaa        480
     Ser Leu Ile Glu Ile Ile Cys Ser Arg Thr Asn Gln Glu Leu Gln Glu
     145                 150                     155                 160

     att aac aga gtc tac aag gaa atg tac aag act gat ctg gag aag gac        528
     Ile Asn Arg Val Tyr Lys Glu Met Tyr Lys Thr Asp Leu Glu Lys Asp
                     165                     170                 175

     att att tcg gac aca tct ggt gac ttc cgc aag ctg atg gtt gcc ctg        576
     Ile Ile Ser Asp Thr Ser Gly Asp Phe Arg Lys Leu Met Val Ala Leu
                     180                     185                 190

     gca aag ggt aga aga gca gag gat ggc tct gtc att gat tat gaa ctg        624
     Ala Lys Gly Arg Arg Ala Glu Asp Gly Ser Val Ile Asp Tyr Glu Leu
                     195                     200                 205

     att gac caa gat gct cgg gat ctc tat gac gct gga gtg aag agg aaa        672
     Ile Asp Gln Asp Ala Arg Asp Leu Tyr Asp Ala Gly Val Lys Arg Lys
             210                     215                     220

     gga act gat gtt ccc aag tgg atc agc atc atg acc gag cgg agc gtg        720
     Gly Thr Asp Val Pro Lys Trp Ile Ser Ile Met Thr Glu Arg Ser Val
     225                     230                     235                 240

     ccc cac ctc cag aaa gta ttt gat agg tac aag agt tac agc cct tat        768
     Pro His Leu Gln Lys Val Phe Asp Arg Tyr Lys Ser Tyr Ser Pro Tyr
                     245                     250                     255

     gac atg ttg gaa agc atc agg aaa gag gtt aaa gga gac ctg gaa aat        816
     Asp Met Leu Glu Ser Ile Arg Lys Glu Val Lys Gly Asp Leu Glu Asn
                     260                     265                     270

     gct ttc ctg aac ctg gtt cag tgc att cag aac aag ccc ctg tat ttt        864
     Ala Phe Leu Asn Leu Val Gln Cys Ile Gln Asn Lys Pro Leu Tyr Phe
                 275                     280                     285

     gct gat cgg ctg tat gac tcc atg aag ggc aag ggg acg cga gat aag        912
     Ala Asp Arg Leu Tyr Asp Ser Met Lys Gly Lys Gly Thr Arg Asp Lys
             290                     295                     300

     gtc ctg atc aga atc atg gtc tcc cgc agt gaa gtg gac atg ttg aaa        960
     Val Leu Ile Arg Ile Met Val Ser Arg Ser Glu Val Asp Met Leu Lys
     305                     310                     315                 320

     att agg tct gaa ttc aag aga aag tac ggc aag tcc ctg tac tat tat       1008
     Ile Arg Ser Glu Phe Lys Arg Lys Tyr Gly Lys Ser Leu Tyr Tyr Tyr
                     325                     330                     335

     atc cag caa gac act aag ggc gac tac cag aaa gcg ctg ctg tac ctg       1056
     Ile Gln Gln Asp Thr Lys Gly Asp Tyr Gln Lys Ala Leu Leu Tyr Leu
                     340                     345                     350

     tgt ggt gga gat gac tga                                              1074
     Cys Gly Gly Asp Asp
                 355


     <210>   4
     <211>   357
     <212>   PRT
     <213>   Homo sapiens
```

<400> 4

Met Gly Arg Gln Leu Ala Gly Cys Gly Asp Ala Gly Lys Lys Ala Ser
1               5                   10                  15

Phe Lys Met Ser Thr Val His Glu Ile Leu Cys Lys Leu Ser Leu Glu
            20                  25                  30

Gly Asp His Ser Thr Pro Pro Ser Ala Tyr Gly Ser Val Lys Ala Tyr
            35                  40                  45

Thr Asn Phe Asp Ala Glu Arg Asp Ala Leu Asn Ile Glu Thr Ala Ile
            50                  55                  60

Lys Thr Lys Gly Val Asp Glu Val Thr Ile Val Asn Ile Leu Thr Asn
65                  70                  75                  80

Arg Ser Asn Ala Gln Arg Gln Asp Ile Ala Phe Ala Tyr Gln Arg Arg
                85                  90                  95

Thr Lys Lys Glu Leu Ala Ser Ala Leu Lys Ser Ala Leu Ser Gly His
            100                 105                 110

Leu Glu Thr Val Ile Leu Gly Leu Leu Lys Thr Pro Ala Gln Tyr Asp
            115                 120                 125

Ala Ser Glu Leu Lys Ala Ser Met Lys Gly Leu Gly Thr Asp Glu Asp
        130                 135                 140

Ser Leu Ile Glu Ile Ile Cys Ser Arg Thr Asn Gln Glu Leu Gln Glu
145                 150                 155                 160

Ile Asn Arg Val Tyr Lys Glu Met Tyr Lys Thr Asp Leu Glu Lys Asp
                165                 170                 175

Ile Ile Ser Asp Thr Ser Gly Asp Phe Arg Lys Leu Met Val Ala Leu
            180                 185                 190

Ala Lys Gly Arg Arg Ala Glu Asp Gly Ser Val Ile Asp Tyr Glu Leu
        195                 200                 205

Ile Asp Gln Asp Ala Arg Asp Leu Tyr Asp Ala Gly Val Lys Arg Lys
        210                 215                 220

Gly Thr Asp Val Pro Lys Trp Ile Ser Ile Met Thr Glu Arg Ser Val
225                 230                 235                 240

27

```
Pro His Leu Gln Lys Val Phe Asp Arg Tyr Lys Ser Tyr Ser Pro Tyr
            245             250             255

Asp Met Leu Glu Ser Ile Arg Lys Glu Val Lys Gly Asp Leu Glu Asn
            260             265             270

Ala Phe Leu Asn Leu Val Gln Cys Ile Gln Asn Lys Pro Leu Tyr Phe
            275             280             285

Ala Asp Arg Leu Tyr Asp Ser Met Lys Gly Lys Gly Thr Arg Asp Lys
            290             295             300

Val Leu Ile Arg Ile Met Val Ser Arg Ser Glu Val Asp Met Leu Lys
305             310             315             320

Ile Arg Ser Glu Phe Lys Arg Lys Tyr Gly Lys Ser Leu Tyr Tyr Tyr
            325             330             335

Ile Gln Gln Asp Thr Lys Gly Asp Tyr Gln Lys Ala Leu Leu Tyr Leu
            340             345             350

Cys Gly Gly Asp Asp
            355


<210>   5
<211>   1020
<212>   DNA
<213>   Homo sapiens


<220>
<221>   CDS
<222>   (1)..(1020)


<400>   5
atg tct act gtt cac gaa atc ctg tgc aag ctc agc ttg gag ggt gat      48
Met Ser Thr Val His Glu Ile Leu Cys Lys Leu Ser Leu Glu Gly Asp
1               5               10              15

cac tct aca ccc cca agt gca tat ggg tct gtc aaa gcc tat act aac      96
His Ser Thr Pro Pro Ser Ala Tyr Gly Ser Val Lys Ala Tyr Thr Asn
            20              25              30

ttt gat gct gag cgg gat gct ttg aac att gaa aca gcc atc aag acc     144
Phe Asp Ala Glu Arg Asp Ala Leu Asn Ile Glu Thr Ala Ile Lys Thr
            35              40              45

aaa ggt gtg gat gag gtc acc att gtc aac att ttg acc aac cgc agc     192
Lys Gly Val Asp Glu Val Thr Ile Val Asn Ile Leu Thr Asn Arg Ser
            50              55              60

aat gca cag aga cag gat att gcc ttc gcc tac cag aga agg acc aaa     240
Asn Ala Gln Arg Gln Asp Ile Ala Phe Ala Tyr Gln Arg Arg Thr Lys
65              70              75              80
```

```
aag gaa ctt gca tca gca ctg aag tca gcc tta tct ggc cac ctg gag        288
Lys Glu Leu Ala Ser Ala Leu Lys Ser Ala Leu Ser Gly His Leu Glu
            85                  90                  95

acg gtg att ttg ggc cta ttg aag aca cct gct cag tat gac gct tct        336
Thr Val Ile Leu Gly Leu Leu Lys Thr Pro Ala Gln Tyr Asp Ala Ser
            100                 105                 110

gag cta aaa gct tcc atg aag ggg ctg gga acc gac gag gac tct ctc        384
Glu Leu Lys Ala Ser Met Lys Gly Leu Gly Thr Asp Glu Asp Ser Leu
            115                 120                 125

att gag atc atc tgc tcc aga acc aac cag gag ctg cag gaa att aac        432
Ile Glu Ile Ile Cys Ser Arg Thr Asn Gln Glu Leu Gln Glu Ile Asn
            130                 135                 140

aga gtc tac aag gaa atg tac aag act gat ctg gag aag gac att att        480
Arg Val Tyr Lys Glu Met Tyr Lys Thr Asp Leu Glu Lys Asp Ile Ile
145                 150                 155                 160

tcg gac aca tct ggt gac ttc cgc aag ctg atg gtt gcc ctg gca aag        528
Ser Asp Thr Ser Gly Asp Phe Arg Lys Leu Met Val Ala Leu Ala Lys
            165                 170                 175

ggt aga aga gca gag gat ggc tct gtc att gat tat gaa ctg att gac        576
Gly Arg Arg Ala Glu Asp Gly Ser Val Ile Asp Tyr Glu Leu Ile Asp
            180                 185                 190

caa gat gct cgg gat ctc tat gac gct gga gtg aag agg aaa gga act        624
Gln Asp Ala Arg Asp Leu Tyr Asp Ala Gly Val Lys Arg Lys Gly Thr
            195                 200                 205

gat gtt ccc aag tgg atc agc atc atg acc gag cgg agc gtg ccc cac        672
Asp Val Pro Lys Trp Ile Ser Ile Met Thr Glu Arg Ser Val Pro His
            210                 215                 220

ctc cag aaa gta ttt gat agg tac aag agt tac agc cct tat gac atg        720
Leu Gln Lys Val Phe Asp Arg Tyr Lys Ser Tyr Ser Pro Tyr Asp Met
225                 230                 235                 240

ttg gaa agc atc agg aaa gag gtt aaa gga gac ctg gaa aat gct ttc        768
Leu Glu Ser Ile Arg Lys Glu Val Lys Gly Asp Leu Glu Asn Ala Phe
            245                 250                 255

ctg aac ctg gtt cag tgc att cag aac aag ccc ctg tat ttt gct gat        816
Leu Asn Leu Val Gln Cys Ile Gln Asn Lys Pro Leu Tyr Phe Ala Asp
            260                 265                 270

cgg ctg tat gac tcc atg aag ggc aag ggg acg cga gat aag gtc ctg        864
Arg Leu Tyr Asp Ser Met Lys Gly Lys Gly Thr Arg Asp Lys Val Leu
            275                 280                 285

atc aga atc atg gtc tcc cgc agt gaa gtg gac atg ttg aaa att agg        912
Ile Arg Ile Met Val Ser Arg Ser Glu Val Asp Met Leu Lys Ile Arg
            290                 295                 300

tct gaa ttc aag aga aag tac ggc aag tcc ctg tac tat tat atc cag        960
Ser Glu Phe Lys Arg Lys Tyr Gly Lys Ser Leu Tyr Tyr Tyr Ile Gln
305                 310                 315                 320

caa gac act aag ggc gac tac cag aaa gcg ctg ctg tac ctg tgt ggt        1008
Gln Asp Thr Lys Gly Asp Tyr Gln Lys Ala Leu Leu Tyr Leu Cys Gly
            325                 330                 335
```

```
gga gat gac tga                                                    1020
Gly Asp Asp
```

<210>  6
<211>  339
<212>  PRT
<213>  Homo sapiens

<400>  6

```
Met Ser Thr Val His Glu Ile Leu Cys Lys Leu Ser Leu Glu Gly Asp
1               5                   10                  15


His Ser Thr Pro Pro Ser Ala Tyr Gly Ser Val Lys Ala Tyr Thr Asn
            20                  25                  30


Phe Asp Ala Glu Arg Asp Ala Leu Asn Ile Glu Thr Ala Ile Lys Thr
        35                  40                  45


Lys Gly Val Asp Glu Val Thr Ile Val Asn Ile Leu Thr Asn Arg Ser
        50                  55                  60


Asn Ala Gln Arg Gln Asp Ile Ala Phe Ala Tyr Gln Arg Arg Thr Lys
65                  70                  75                  80


Lys Glu Leu Ala Ser Ala Leu Lys Ser Ala Leu Ser Gly His Leu Glu
                85                  90                  95


Thr Val Ile Leu Gly Leu Leu Lys Thr Pro Ala Gln Tyr Asp Ala Ser
                100                 105                 110


Glu Leu Lys Ala Ser Met Lys Gly Leu Gly Thr Asp Glu Asp Ser Leu
            115                 120                 125


Ile Glu Ile Ile Cys Ser Arg Thr Asn Gln Glu Leu Gln Glu Ile Asn
        130                 135                 140


Arg Val Tyr Lys Glu Met Tyr Lys Thr Asp Leu Glu Lys Asp Ile Ile
145                 150                 155                 160


Ser Asp Thr Ser Gly Asp Phe Arg Lys Leu Met Val Ala Leu Ala Lys
                165                 170                 175


Gly Arg Arg Ala Glu Asp Gly Ser Val Ile Asp Tyr Glu Leu Ile Asp
            180                 185                 190


Gln Asp Ala Arg Asp Leu Tyr Asp Ala Gly Val Lys Arg Lys Gly Thr
            195                 200                 205
```

```
Asp Val Pro Lys Trp Ile Ser Ile Met Thr Glu Arg Ser Val Pro His
    210                 215                 220

Leu Gln Lys Val Phe Asp Arg Tyr Lys Ser Tyr Ser Pro Tyr Asp Met
    225                 230                 235                 240

Leu Glu Ser Ile Arg Lys Glu Val Lys Gly Asp Leu Glu Asn Ala Phe
                    245                 250                 255

Leu Asn Leu Val Gln Cys Ile Gln Asn Lys Pro Leu Tyr Phe Ala Asp
                260                 265                 270

Arg Leu Tyr Asp Ser Met Lys Gly Lys Gly Thr Arg Asp Lys Val Leu
            275                 280                 285

Ile Arg Ile Met Val Ser Arg Ser Glu Val Asp Met Leu Lys Ile Arg
        290                 295                 300

Ser Glu Phe Lys Arg Lys Tyr Gly Lys Ser Leu Tyr Tyr Tyr Ile Gln
305                 310                 315                 320

Gln Asp Thr Lys Gly Asp Tyr Gln Lys Ala Leu Leu Tyr Leu Cys Gly
                325                 330                 335

Gly Asp Asp


<210>   7
<211>   1020
<212>   DNA
<213>   Homo sapiens


<220>
<221>   CDS
<222>   (1)..(1020)

<400>   7
atg tct act gtt cac gaa atc ctg tgc aag ctc agc ttg gag ggt gat       48
Met Ser Thr Val His Glu Ile Leu Cys Lys Leu Ser Leu Glu Gly Asp
1               5                   10                  15

cac tct aca ccc cca agt gca tat ggg tct gtc aaa gcc tat act aac       96
His Ser Thr Pro Pro Ser Ala Tyr Gly Ser Val Lys Ala Tyr Thr Asn
                20                  25                  30

ttt gat gct gag cgg gat gct ttg aac att gaa aca gcc atc aag acc      144
Phe Asp Ala Glu Arg Asp Ala Leu Asn Ile Glu Thr Ala Ile Lys Thr
            35                  40                  45

aaa ggt gtg gat gag gtc acc att gtc aac att ttg acc aac cgc agc      192
Lys Gly Val Asp Glu Val Thr Ile Val Asn Ile Leu Thr Asn Arg Ser
```

31

```
              50                        55                        60

    aat gca cag aga cag gat att gcc ttc gcc tac cag aga agg acc aaa     240
    Asn Ala Gln Arg Gln Asp Ile Ala Phe Ala Tyr Gln Arg Arg Thr Lys
    65                  70                  75                  80

    aag gaa ctt gca tca gca ctg aag tca gcc tta tct ggc cac ctg gag     288
    Lys Glu Leu Ala Ser Ala Leu Lys Ser Ala Leu Ser Gly His Leu Glu
                    85                  90                  95

    acg gtg att ttg ggc cta ttg aag aca cct gct cag tat gac gct tct     336
    Thr Val Ile Leu Gly Leu Leu Lys Thr Pro Ala Gln Tyr Asp Ala Ser
                    100                 105                 110

    gag cta aaa gct tcc atg aag ggg ctg gga acc gac gag gac tct ctc     384
    Glu Leu Lys Ala Ser Met Lys Gly Leu Gly Thr Asp Glu Asp Ser Leu
                    115                 120                 125

    att gag atc atc tgc tcc aga acc aac cag gag ctg cag gaa att aac     432
    Ile Glu Ile Ile Cys Ser Arg Thr Asn Gln Glu Leu Gln Glu Ile Asn
                    130                 135                 140

    aga gtc tac aag gaa atg tac aag act gat ctg gag aag gac att att     480
    Arg Val Tyr Lys Glu Met Tyr Lys Thr Asp Leu Glu Lys Asp Ile Ile
    145                 150                 155                 160

    tcg gac aca tct ggt gac ttc cgc aag ctg atg gtt gcc ctg gca aag     528
    Ser Asp Thr Ser Gly Asp Phe Arg Lys Leu Met Val Ala Leu Ala Lys
                    165                 170                 175

    ggt aga aga gca gag gat ggc tct gtc att gat tat gaa ctg att gac     576
    Gly Arg Arg Ala Glu Asp Gly Ser Val Ile Asp Tyr Glu Leu Ile Asp
                    180                 185                 190

    caa gat gct cgg gat ctc tat gac gct gga gtg aag agg aaa gga act     624
    Gln Asp Ala Arg Asp Leu Tyr Asp Ala Gly Val Lys Arg Lys Gly Thr
                    195                 200                 205

    gat gtt ccc aag tgg atc agc atc atg acc gag cgg agc gtg ccc cac     672
    Asp Val Pro Lys Trp Ile Ser Ile Met Thr Glu Arg Ser Val Pro His
                    210                 215                 220

    ctc cag aaa gta ttt gat agg tac aag agt tac agc cct tat gac atg     720
    Leu Gln Lys Val Phe Asp Arg Tyr Lys Ser Tyr Ser Pro Tyr Asp Met
    225                 230                 235                 240

    ttg gaa agc atc agg aaa gag gtt aaa gga gac ctg gaa aat gct ttc     768
    Leu Glu Ser Ile Arg Lys Glu Val Lys Gly Asp Leu Glu Asn Ala Phe
                    245                 250                 255

    ctg aac ctg gtt cag tgc att cag aac aag ccc ctg tat ttt gct gat     816
    Leu Asn Leu Val Gln Cys Ile Gln Asn Lys Pro Leu Tyr Phe Ala Asp
                    260                 265                 270

    cgg ctg tat gac tcc atg aag ggc aag ggg acg cga gat aag gtc ctg     864
    Arg Leu Tyr Asp Ser Met Lys Gly Lys Gly Thr Arg Asp Lys Val Leu
                    275                 280                 285

    atc aga atc atg gtc tcc cgc agt gaa gtg gac atg ttg aaa att agg     912
    Ile Arg Ile Met Val Ser Arg Ser Glu Val Asp Met Leu Lys Ile Arg
                    290                 295                 300

    tct gaa ttc aag aga aag tac ggc aag tcc ctg tac tat tat atc cag     960
```

```
Ser Glu Phe Lys Arg Lys Tyr Gly Lys Ser Leu Tyr Tyr Tyr Ile Gln
305             310             315             320


caa gac act aag ggc gac tac cag aaa gcg ctg ctg tac ctg tgt ggt          1008
Gln Asp Thr Lys Gly Asp Tyr Gln Lys Ala Leu Leu Tyr Leu Cys Gly
                325             330             335


gga gat gac tga                                                          1020
Gly Asp Asp



<210>   8
<211>   339
<212>   PRT
<213>   Homo sapiens


<400>   8

Met Ser Thr Val His Glu Ile Leu Cys Lys Leu Ser Leu Glu Gly Asp
1               5               10              15


His Ser Thr Pro Pro Ser Ala Tyr Gly Ser Val Lys Ala Tyr Thr Asn
            20              25              30


Phe Asp Ala Glu Arg Asp Ala Leu Asn Ile Glu Thr Ala Ile Lys Thr
        35              40              45


Lys Gly Val Asp Glu Val Thr Ile Val Asn Ile Leu Thr Asn Arg Ser
    50              55              60


Asn Ala Gln Arg Gln Asp Ile Ala Phe Ala Tyr Gln Arg Arg Thr Lys
65              70              75              80


Lys Glu Leu Ala Ser Ala Leu Lys Ser Ala Leu Ser Gly His Leu Glu
            85              90              95


Thr Val Ile Leu Gly Leu Leu Lys Thr Pro Ala Gln Tyr Asp Ala Ser
            100             105             110


Glu Leu Lys Ala Ser Met Lys Gly Leu Gly Thr Asp Glu Asp Ser Leu
        115             120             125


Ile Glu Ile Ile Cys Ser Arg Thr Asn Gln Glu Leu Gln Glu Ile Asn
    130             135             140


Arg Val Tyr Lys Glu Met Tyr Lys Thr Asp Leu Glu Lys Asp Ile Ile
145             150             155             160


Ser Asp Thr Ser Gly Asp Phe Arg Lys Leu Met Val Ala Leu Ala Lys
            165             170             175
```

33

```
Gly Arg Arg Ala Glu Asp Gly Ser Val Ile Asp Tyr Glu Leu Ile Asp
            180                 185                 190

Gln Asp Ala Arg Asp Leu Tyr Asp Ala Gly Val Lys Arg Lys Gly Thr
            195                 200                 205

Asp Val Pro Lys Trp Ile Ser Ile Met Thr Glu Arg Ser Val Pro His
    210                 215                 220

Leu Gln Lys Val Phe Asp Arg Tyr Lys Ser Tyr Ser Pro Tyr Asp Met
225                 230                 235                 240

Leu Glu Ser Ile Arg Lys Glu Val Lys Gly Asp Leu Glu Asn Ala Phe
                245                 250                 255

Leu Asn Leu Val Gln Cys Ile Gln Asn Lys Pro Leu Tyr Phe Ala Asp
            260                 265                 270

Arg Leu Tyr Asp Ser Met Lys Gly Lys Gly Thr Arg Asp Lys Val Leu
        275                 280                 285

Ile Arg Ile Met Val Ser Arg Ser Glu Val Asp Met Leu Lys Ile Arg
    290                 295                 300

Ser Glu Phe Lys Arg Lys Tyr Gly Lys Ser Leu Tyr Tyr Tyr Ile Gln
305                 310                 315                 320

Gln Asp Thr Lys Gly Asp Tyr Gln Lys Ala Leu Leu Tyr Leu Cys Gly
                325                 330                 335

Gly Asp Asp
```

```
<210>  9
<211>  1020
<212>  DNA
<213>  Rattus norvegicus

<220>
<221>  CDS
<222>  (1)..(1020)

<400>  9
atg tct act gtc cac gaa atc ctg tgc aag ctc agc ttg gag ggt gat        48
Met Ser Thr Val His Glu Ile Leu Cys Lys Leu Ser Leu Glu Gly Asp
1               5                   10                  15

cat tct aca ccc cca agt gcc tat ggg tcg gtc aaa ccc tac acc aac        96
His Ser Thr Pro Pro Ser Ala Tyr Gly Ser Val Lys Pro Tyr Thr Asn
                20                  25                  30
```

```
ttc gac gct gag agg gat gct ttg aac att gaa aca gca atc aag acc        144
Phe Asp Ala Glu Arg Asp Ala Leu Asn Ile Glu Thr Ala Ile Lys Thr
        35              40              45

aaa ggc gtg gac gag gtc acc att gtc aac att ctg act aac cgc agc        192
Lys Gly Val Asp Glu Val Thr Ile Val Asn Ile Leu Thr Asn Arg Ser
        50              55              60

aat gca cag agg cag gac att gcc ttc gcc tac cag agg agg acc aaa        240
Asn Ala Gln Arg Gln Asp Ile Ala Phe Ala Tyr Gln Arg Arg Thr Lys
65              70              75              80

aag gaa ctg cca tcg gcg atg aag tcg gcc ttg tct ggt cac ctg gag        288
Lys Glu Leu Pro Ser Ala Met Lys Ser Ala Leu Ser Gly His Leu Glu
                85              90              95

acc gtg atg tta ggc ctg ttg aag aca cct gct cag tac gat gcc tct        336
Thr Val Met Leu Gly Leu Leu Lys Thr Pro Ala Gln Tyr Asp Ala Ser
            100             105             110

gag ctc aaa gcc tcc atg aag ggc ctg ggg act gat gag gac tcc ctc        384
Glu Leu Lys Ala Ser Met Lys Gly Leu Gly Thr Asp Glu Asp Ser Leu
            115             120             125

atc gag atc atc tgc tca aga acc aac cag gag ctg cag gag att aac        432
Ile Glu Ile Ile Cys Ser Arg Thr Asn Gln Glu Leu Gln Glu Ile Asn
        130             135             140

cga gtg tat aag gaa atg tac aag acc gat ctg gag aag gac atc atc        480
Arg Val Tyr Lys Glu Met Tyr Lys Thr Asp Leu Glu Lys Asp Ile Ile
145             150             155             160

tct gac aca tct gga gaa ttc cga aag ctg ttg gtc gcc ctt gca aag        528
Ser Asp Thr Ser Gly Glu Phe Arg Lys Leu Leu Val Ala Leu Ala Lys
                165             170             175

ggt aaa cgg gca gag gat ggt tct gtt att gac tac gag ctg att gac        576
Gly Lys Arg Ala Glu Asp Gly Ser Val Ile Asp Tyr Glu Leu Ile Asp
            180             185             190

cag gat gcc cgg gag ctc tat gat gct ggg gtg aag agg aaa gga acc        624
Gln Asp Ala Arg Glu Leu Tyr Asp Ala Gly Val Lys Arg Lys Gly Thr
            195             200             205

gat gtc ccc aag tgg atc agc atc atg act gag cgc agt gtg tgc cac        672
Asp Val Pro Lys Trp Ile Ser Ile Met Thr Glu Arg Ser Val Cys His
        210             215             220

ctc cag aaa gtg ttc gaa agg tac aag agc tac agt cct tat gac atg        720
Leu Gln Lys Val Phe Glu Arg Tyr Lys Ser Tyr Ser Pro Tyr Asp Met
225             230             235             240

ctg gag agc atc agg aaa gag gtc aaa gga gac ctg gag aac gcc ttc        768
Leu Glu Ser Ile Arg Lys Glu Val Lys Gly Asp Leu Glu Asn Ala Phe
                245             250             255

ctg aac ctg gtt cag tgc att cag aac aag ccc ctg tac ttt gct gac        816
Leu Asn Leu Val Gln Cys Ile Gln Asn Lys Pro Leu Tyr Phe Ala Asp
            260             265             270

cgg ctg tat gac tcc atg aag ggc aag ggg act cga gac aag gtc ctg        864
Arg Leu Tyr Asp Ser Met Lys Gly Lys Gly Thr Arg Asp Lys Val Leu
            275             280             285
```

```
att aga atc atg gtc tct cgc agt gaa gtg gac atg ttg aaa atc aga          912
Ile Arg Ile Met Val Ser Arg Ser Glu Val Asp Met Leu Lys Ile Arg
    290             295             300

tct gaa ttc aag agg aaa tat ggc aaa tcc ctg tac tac ttc atc cag          960
Ser Glu Phe Lys Arg Lys Tyr Gly Lys Ser Leu Tyr Tyr Phe Ile Gln
305             310             315             320

caa gac act aag ggt gac tac cag aag gcg ctg ctg tac ctg tgt ggt         1008
Gln Asp Thr Lys Gly Asp Tyr Gln Lys Ala Leu Leu Tyr Leu Cys Gly
            325             330             335

ggg gac gac tga                                                         1020
Gly Asp Asp
```

<210> 10
<211> 339
<212> PRT
<213> Rattus norvegicus

<400> 10

```
Met Ser Thr Val His Glu Ile Leu Cys Lys Leu Ser Leu Glu Gly Asp
1               5               10              15

His Ser Thr Pro Pro Ser Ala Tyr Gly Ser Val Lys Pro Tyr Thr Asn
            20              25              30

Phe Asp Ala Glu Arg Asp Ala Leu Asn Ile Glu Thr Ala Ile Lys Thr
            35              40              45

Lys Gly Val Asp Glu Val Thr Ile Val Asn Ile Leu Thr Asn Arg Ser
        50              55              60

Asn Ala Gln Arg Gln Asp Ile Ala Phe Ala Tyr Gln Arg Arg Thr Lys
65              70              75              80

Lys Glu Leu Pro Ser Ala Met Lys Ser Ala Leu Ser Gly His Leu Glu
                85              90              95

Thr Val Met Leu Gly Leu Leu Lys Thr Pro Ala Gln Tyr Asp Ala Ser
            100             105             110

Glu Leu Lys Ala Ser Met Lys Gly Leu Gly Thr Asp Glu Asp Ser Leu
            115             120             125

Ile Glu Ile Ile Cys Ser Arg Thr Asn Gln Glu Leu Gln Glu Ile Asn
        130             135             140

Arg Val Tyr Lys Glu Met Tyr Lys Thr Asp Leu Glu Lys Asp Ile Ile
145             150             155             160
```

```
Ser Asp Thr Ser Gly Glu Phe Arg Lys Leu Leu Val Ala Leu Ala Lys
                165             170             175

Gly Lys Arg Ala Glu Asp Gly Ser Val Ile Asp Tyr Glu Leu Ile Asp
            180             185             190

Gln Asp Ala Arg Glu Leu Tyr Asp Ala Gly Val Lys Arg Lys Gly Thr
            195             200             205

Asp Val Pro Lys Trp Ile Ser Ile Met Thr Glu Arg Ser Val Cys His
    210             215             220

Leu Gln Lys Val Phe Glu Arg Tyr Lys Ser Tyr Ser Pro Tyr Asp Met
225             230             235             240

Leu Glu Ser Ile Arg Lys Glu Val Lys Gly Asp Leu Glu Asn Ala Phe
            245             250             255

Leu Asn Leu Val Gln Cys Ile Gln Asn Lys Pro Leu Tyr Phe Ala Asp
            260             265             270

Arg Leu Tyr Asp Ser Met Lys Gly Lys Gly Thr Arg Asp Lys Val Leu
            275             280             285

Ile Arg Ile Met Val Ser Arg Ser Glu Val Asp Met Leu Lys Ile Arg
    290             295             300

Ser Glu Phe Lys Arg Lys Tyr Gly Lys Ser Leu Tyr Tyr Phe Ile Gln
305             310             315             320

Gln Asp Thr Lys Gly Asp Tyr Gln Lys Ala Leu Leu Tyr Leu Cys Gly
            325             330             335

Gly Asp Asp
```

**Description**

BACKGROUND OF THE INVENTION

Field of the Invention

The present invention relates to: a method and kit for assisting diagnosis of a disease in a subject using a disease marker and a reference marker; and a method and kit for collecting a disease marker and a reference marker from a subject to assist diagnosis of a disease in a subject.

Background Art

In a conventionally known method, albumin existing in interstitial fluid of a subject is transcutaneously and noninvasively collected using a sheet capable of generating an attractive force due to electrostatic interaction (Patent Literature 1).
In addition, it is known that a disease marker serving as an index for a disease can be transcutaneously collected (Non-Patent Literature 1 to 15).
In this regard, the skin is divided into the epidermis and dermis in this order from the surface layer. The epidermis is divided into the stratum corneum, stratum granulosum, stratum spinosum and stratum basale, and the dermis is divided into the stratum papillare and stratum reticulare. A layer of subcutaneous adipose tissue underlies the skin, and at many sites, a layer of skeletal muscle underlies the subcutaneous adipose tissue. A skin barrier function that inhibits leakage of moisture or electrolyte, invasion of foreign matter, and the like is served mainly by the sebum barrier of the skin surface, the intercorneocyte lipid of the stratum corneum, the tight junction of the stratum granulosum, and the like.
As mentioned above, the skin has a barrier function, and thus, when transcutaneously collecting a disease marker existing in interstitial fluid of a layer located below a skin surface of a subject (in particular, a disease marker existing in interstitial fluid of a layer (for example, one or more layers selected from the stratum spinosum and stratum basale of the epidermis, the dermis, the subcutaneous fat, the skeletal muscle, and the like) located below a layer serving a skin barrier function (the sebum barrier of the skin surface, the stratum corneum and stratum granulosum of the epidermis, and the like)), variation in the amount of the collected disease marker can be caused by variation in the skin barrier function.

Citation List

Patent Literature

Patent Literature 1: JP2018-048984A

Non-Patent Literature

Non-Patent Literature 1: Minematsu et al., ADVANCES IN SKIN & WOUND CARE 2014; 27: 272-9
Non-Patent Literature 2: Ogai et al., International Journal of Cosmetic Science, 2015, 27, 425-432
Non-Patent Literature 3: Ogai et al., International Journal of Cosmetic Science, 2016, 38, 462-469
Non-Patent Literature 4: Kishi et al., Biological Research for Nursing 2015, Vol. 17(2) 135-141
Non-Patent Literature 5: Koyano et al., International Wound Journal, 2016, Vol. 13(2) 189-97
Non-Patent Literature 6: Tamai et al., Journal of Nursing Science and Engineering, 2017, Vol. 4(2) 116-120
Non-Patent Literature 7: Kaneko et al., Wound Repair and Regeneration, 2015, Vol. 23, 657-663
Non-Patent Literature 8: Kanazawa et al., PLOS ONE, 2014, Vol. 9(8) 1-11
Non-Patent Literature 9: Kimura, Master's Thesis; Division of Health Sciences and Nursing, Graduate School of Medicine, The University of Tokyo; 2016
Non-Patent Literature 10: Kimura et al., Abstract of Oral Presentation at the 46th Annual Meeting of the Japanese Society for Wound Healing
Non-Patent Literature 11: Nakai, Master's Thesis; Division of Health Sciences and Nursing, Graduate School of Medicine, The University of Tokyo; 2018
Non-Patent Literature 12: Neya et al., Abstract of the 70th AnnualMeeting of the Japanese Society of Physical Fitness and Sports Medicine
Non-Patent Literature 13: Neya et al., Abstract of the 72nd Annual Meeting of the Japanese Society of Physical Fitness and Sports Medicine
Non-Patent Literature 14: Sari et al., WOUNDS, 2010, Vol. 22(2), 44-51
Non-Patent Literature 15: Minematsu et al., Abstract of the 19th Annual Meeting of Japan Society of Clinical Hair Restoration

## SUMMARY OF THE INVENTION

Technical Problem

When transcutaneously collecting a disease marker existing in interstitial fluid of a layer located below a skin surface of a subject (for example, one or more layers selected from the stratum spinosum and stratum basale of the epidermis, the dermis, the subcutaneous fat, the skeletal muscle, and the like), variation in the amount of the collected disease marker can be caused by variation in the skin barrier function of the subject, and thus leads to a problem that the amount of the collected disease marker can fail to reflect the actual health condition of the subject accurately. For example, when the skin barrier function of the subject is lower than in a healthy state, the disease marker tends more to pass through the skin barrier than in the healthy state, and the amount of the disease marker transcutaneously collected increases, thus failing to allow the amount of the collected disease marker to reflect the actual health condition of the subject accurately. On the other hand, when the skin barrier function of the subject is higher than in a healthy state, the disease marker tends less to pass through the skin barrier, and the amount of the disease marker transcutaneously collected becomes lower than in the healthy state, thus failing to allow the amount of the collected disease marker to reflect the actual health condition of the subject accurately.

An object of the present invention is to provide: a method and kit for assisting diagnosis of the health condition of a subject by correcting variation in an amount of a collected disease marker, which is caused by variation in the skin barrier function of the subject, and thus acquiring information reflecting the health condition of the subject accurately; and a method and kit for collecting a disease marker and a reference marker from a subject to assist diagnosis of the health condition of the subject.

Solution to Problem

The present inventors have accomplished the present invention, based on the findings that:

when a sheet capable of generating an attractive force due to electrostatic interaction between the sheet and each of disease and reference markers in interstitial fluid existing in a layer located below a skin surface of a subject (for example, one or more layers selected from the stratum spinosum and stratum basale of the epidermis, the dermis, the subcutaneous fat, the skeletal muscle, and the like) is applied to the skin surface of the subject, the attractive force due to electrostatic interaction between the sheet and each of the disease and reference markers is generated, and thus the disease and reference markers in interstitial fluid of the subject are attracted to the sheet by passing through the epidermis of the subject, and are attached to the surface of the sheet;
when the sheet, to the surface of which the disease and reference markers are attached, is detached from the skin surface, the detachment of the sheet from the skin surface makes it possible to collect the disease and reference markers in a state where the disease and reference markers are attached to the sheet; and
use of annexin A2 as a reference marker to correct the amount of the collected disease marker with the amount of the collected reference marker makes it possible to obtain information reflecting the actual health condition more accurately than information on the health condition obtained based on the amount of the disease marker alone, and thus enhance the accuracy of diagnosis.

Thus, the present invention encompasses the following inventions.

[1] A method of assisting diagnosis of a disease in a subject using a disease marker and a reference marker, the method comprising the following steps of:

(a) providing a sheet capable of generating an attractive force due to electrostatic interaction between the sheet and each of the disease and reference markers;
(b) applying the sheet to a skin surface of the subject, followed by detaching the sheet from the skin surface;
(c) measuring an amount of each of the disease and reference markers that are attached to the sheet; and
(d) acquiring information on the disease in the subject by using a value obtained by correcting the amount of the disease marker measured in step (c) with the amount of the reference marker measured in step (c);

wherein the reference marker is annexin A2.

[2] A method of collecting a disease marker and a reference marker from a subject to assist diagnosis of a disease in the subject using the disease marker and the reference marker,
the method comprising the following steps of:

(a) providing a sheet capable of generating an attractive force due to electrostatic interaction between the sheet and each of the disease and reference markers; and
(b) applying the sheet to a skin surface of the subject, followed by detaching the sheet from the skin surface;

wherein the reference marker is annexin A2.

[3] The method according to [1] or [2], wherein the disease marker is a protein selected from the group consisting of cytokines, enzymes, extracellular matrix proteins, plasma proteins, heat-shock proteins, and receptor proteins, or is an osmolyte.

[4] The method according to any one of [1] to [3], wherein the disease marker is a protein selected from the group consisting of TNF$\alpha$, IL1$\alpha$, NGF$\beta$, VEGF-C, TGF$\beta$1, BMP1, PAI1, MMP2, creatine kinase, creatine phosphokinase, COL4, fibronectin, albumin, HSP90$\alpha$, and NOTCH1, or is taurine.

[5] The method according to any one of [1] to [4], wherein the sheet has a polar functional group on a surface thereof.

[6] The method according to [5], wherein the polar functional group is a nitro group.

[7] The method according to [6], wherein the sheet is a nitrocellulose membrane.

[8] The method according to [7], wherein the nitrocellulose membrane has a nitration ratio of 30% or more.

[9] The method according to [5], wherein the polar functional group is a cationic functional group.

[10] The method according to [5], wherein the polar functional group is an anionic functional group.

[11] The method according to any one of [1] to [10], wherein, in step (b), one or both of the sheet and the skin surface is/are wetted with an aqueous medium, and then, the sheet is applied to the skin surface.

[12] A kit for assisting diagnosis of a disease in a subject using a disease marker and a reference marker,
the kit comprising
a sheet capable of generating an attractive force due to electrostatic interaction between the sheet and each of the disease and reference markers,
wherein the reference marker is annexin A2.

[13] A kit for collecting a disease marker and a reference marker from a subject to assist diagnosis of a disease in the subject using the disease marker and the reference marker,
the kit comprising
a sheet capable of generating an attractive force due to electrostatic interaction between the sheet and each of the disease and reference markers,
wherein the reference marker is annexin A2.

[14] The kit according to [12] or [13], wherein the disease marker is a protein selected from the group consisting of cytokines, enzymes, extracellular matrix proteins, plasma proteins, heat-shock proteins, and receptor proteins, or is an osmolyte.

[15] The kit according to any one of [12] to [14], wherein the disease marker is a protein selected from the group consisting of TNF$\alpha$, IL1$\alpha$, NGF$\beta$, VEGF-C, TGF$\beta$1, BMP1, PAI1, MMP2, creatine kinase, creatine phosphokinase, COL4, fibronectin, albumin, HSP90$\alpha$, and NOTCH1, or is taurine.

[16] The kit according to any one of [12] to [15], wherein the sheet has a polar functional group on a surface thereof.

[17] The kit according to [16], wherein the polar functional group is a nitro group.

[18] The kit according to [17], wherein the sheet is a nitrocellulose membrane.

[19] The kit according to [18], wherein the nitrocellulose membrane has a nitration ratio of 30% or more.

[20] The kit according to [16], wherein the polar functional group is a cationic functional group.

[21] The kit according to [16], wherein the polar functional group is an anionic functional group.

[22] The kit according to any one of [12] to [21], further comprising a first reagent for detecting the disease marker and a second reagent for detecting the reference marker.

Advantageous Effects of the Invention

According to the present invention, there are provided a method and kit for assisting diagnosis of the health condition of a subject by correcting variation in an amount of a collected disease marker, which is caused by variation in the skin barrier function of the subject, and thus acquiring information reflecting the health condition of the subject accurately, and a method and kit for collecting a disease marker and a reference marker from a subject to assist diagnosis of the health condition of the subject.

EP 3 812 770 A1

## BRIEF DESCRIPTION OF THE DRAWINGS

FIG. 1 is a graph depicting the verification results on the validity of model animals.
FIG. 2 is an immunostaining photograph of a tissue sample collected from a young rat.
FIG. 3A shows immunostaining photographs of the skin blotting samples collected from four types of rats and immunostained with an anti-annexin A2 antibody. FIG. 3B is a graph showing the measurement results of the alkaline phosphatase luminescence intensity of the skin blotting samples in FIG. 3A.

## DETAILED DESCRIPTION OF THE INVENTION

Below, the present invention will be described.

<Explanation of Terms>

Below, the terms used herein will be explained. The following explanations of the terms are applied throughout the present specification unless otherwise specified.
A "subject" is an animal subjected to diagnosis of a disease. A subject animal is not limited to any particular animal as long as the subject animal has a disease marker and a reference marker in interstitial fluid of a layer located below a skin surface of the subject animal (for example, one or more layers selected from the stratum spinosum and stratum basale of the epidermis, the dermis, the subcutaneous fat, the skeletal muscle, and the like). Examples of subject animals include humans or other mammalian animals (for example, mice, rats, dogs, cats, bovines, horses, pigs, and the like), birds such as domestic fowls and parakeets, and the like.
A "skin" is not limited to any particular skin of a subject as long as both of a disease marker and a reference marker exist in interstitial fluid of a layer located below the skin surface (for example, one or more layers selected from the stratum spinosum and stratum basale of the epidermis, the dermis, the subcutaneous fat, the skeletal muscle, and the like), and the disease and reference markers existing in the interstitial fluid can be transcutaneously collected by applying the below-mentioned sheet to the skin surface. Examples of skins include the skin of a foot, leg, head, hand, arm, face, other body area, or the like.
A "disease" is not limited to any particular disease as long as the disease is diagnosable with a disease marker as an index. Such diseases encompass symptoms, disorders, abnormalities, and the like. Examples of such diseases include inflammation, pruritus, skin tear, decreased skin barrier function, ischemic disease, hypoxic condition, cellular deformation, pressure ulcer/pressure injury, muscle injury, hair cycle abnormality, dehydration, and the like. Such a disease may be local or systemic, and a site where a disease may exist is not limited to any particular site. Examples of such sites where a disease may exist include skins, subcutaneous tissues, and the like.
Among the above-mentioned diseases, inflammation, pruritus, ischemic disease, hypoxic condition, and the like can be diagnosed using, for example, a cytokine as an index. Skin tear can be diagnosed using, for example, a cytokine, an enzyme, an extracellular matrix protein, or the like as an index. Decreased skin barrier function can be diagnosed using, for example, a plasma protein or the like as an index. Cellular deformation can be diagnosed using, for example, a heat-shock protein or the like as an index. Pressure ulcer/pressure injury can be diagnosed using, for example, a cytokine, a heat-shock protein, an enzyme, or the like as an index. Muscle injury can be diagnosed using, for example, an enzyme or the like as an index. Hair cycle abnormality can be diagnosed using, for example, a receptor protein, a cytokine, or the like as an index. Dehydration can be diagnosed using, for example, an osmolyte or the like as an index.
In an embodiment, a disease to be diagnosed is a skin disease.
Examples of skin diseases include dermatitis/eczema, prurigo, erythema, acquired keratosis, disorders of hairs, acute pyodermas, fungal diseases, tumor, injury/trauma/wound/burn, and the like. In the expression "a disease A/a disease B", "/" means "or", and the diseases A and B have different names but the scope of the diseases is the same or almost the same. The same shall apply hereinafter.
Examples of dermatitis/eczema include contact dermatitis, atopic dermatitis, seborrheic dermatitis/seborrhoic eczema, autosensitization dermatitis, stasis dermatitis, and the like. Contact dermatitis encompasses incontinence-associated dermatitis.
Examples of prurigo include skin pruritus and the like.
Examples of erythema include Stevens-Johnson syndrome/mucocutaneous ocular syndrome/EM major and the like.
Examples of acquired keratosis include psoriasis and the like.
Examples of disorders of hairs include alopecia areata, male pattern baldness/androgenic alopecia/alopecia

5

prematura, and the like.

Examples of acute pyoderma include cellulitis, folliculitis, and the like.

Examples of fungal diseases include tinea/dermatophytosis and the like.

Examples of tumor include basal cell carcinoma/basal cell epithelioma, squamous cell carcinoma, actinic keratosis/senile keratosis/solar keratosis, Bowen's disease, metastatic carcinoma of the skin/malignant wound, (malignant) melanoma, and the like.

Examples of injury/trauma/wound/burn include laceration, pressure ulcer/pressure injury, and the like. Laceration encompasses skin tear.

In another embodiment, a disease to be diagnosed is a local disorder other than in a skin.

Examples of local disorders other than in a skin include damage of skeletal muscle and the like.

In still another embodiment, a disease to be diagnosed is a systemic disease or a systemic symptom.

Examples of systemic diseases or systemic symptoms include malnutrition, hypertonic dehydration, and the like.

A "disease marker" is not limited to any particular substance as long as the disease marker is a substance:

that can be an index for determining the current health condition of a subject (for example, the presence or absence of a disease, the severity of a disease, the likelihood of contracting a disease, or the like), the future health condition of a subject (for example, the prognosis of a disease and the like), and the like;

that exists in interstitial fluid of a layer located below a skin surface of the subject (for example, one or more layers selected from the stratum spinosum and stratum basale of the epidermis, the dermis, the subcutaneous fat, the skeletal muscle, and the like); and

that can generate an attractive force due to electrostatic interaction between the below-mentioned sheet and the substance, and thus can be attached to the sheet so as to be collected transcutaneously. A disease marker can suitably be selected with reference to a disease to be diagnosed. Such a disease marker may be a marker which is expressed in a subject when the subject is suffering from a disease but not expressed in the subject when the subject is not suffering from the disease, or may be a marker which is not only expressed in a subject when the subject is suffering from a disease but also expressed in the subject when the subject is not suffering from the disease, and of which the expression amount when the subject is suffering from the disease is different from the expression amount of the marker when the subject is not suffering from the disease (for example, a marker, the expression amount of which when a subject is suffering from a disease is larger or smaller than the expression amount of the marker when the subject is not suffering from the disease). Examples of disease markers include proteins such as cytokines, enzymes, extracellular matrix proteins, plasma proteins, heat-shock proteins, and receptor proteins, and the like. Examples of cytokines include tumor necrosis factor $\alpha$ (TNF$\alpha$), interleukin 1$\alpha$ (IL1$\alpha$), nerve growth factor $\beta$ (NGF$\beta$), vascular endothelial growth factor C (VEGF-C), transforming growth factor $\beta$ (TGF$\beta$1), bone morphogenetic protein 1 (BMP1), plasminogen activation inhibitor 1 (PAI1), and the like. Examples of enzymes include secretase, and specific examples include matrix metalloproteinase 2 (MMP2), creatine kinase (CK), creatine phosphokinase, and the like. Examples of extracellular matrix proteins include type IV collagen (COL4), fibronectin (FN), and the like. Examples of plasma proteins include albumin (ALB) and the like. Examples of heat-shock proteins include heat-shock protein 90a (HSP90a) and the like. Examples of receptor proteins include NOTCH1 and the like.

In addition, examples of disease markers include an osmolyte having a function that regulates osmotic pressure in vivo. Examples of osmolytes include organic osmolytes and the like, and examples of organic osmolytes include amino acids, amino-acid analogs, and the like. Examples of amino-acid analogs include taurine and the like.

In an embodiment, a disease to be diagnosed is contact dermatitis, and a disease marker to be used is a marker for contact dermatitis. Examples of markers for contact dermatitis include trypsin, chymotrypsin, lipase, and the like (Mugita et al., PLOS One 10: e0138117. 2015; Mugita et al., Int Wound J. 15: 623-32, 2018).

In another embodiment, a disease to be diagnosed is atopic dermatitis, and a disease marker to be used is a marker for atopic dermatitis. Examples of markers for atopic dermatitis include IL22, TSLP, IL-1$\alpha$, IL-1$\beta$, IL-18, IL-1RA, IL-5, IL-13, IL-6, IL-8, IL-9, IL-17, IL-22, TNF-a, CCL-5, CXCL-9, CXCL-10, CCL-17, and the like (Zhu et al., Sci Rep 1:23, 2011; Szegedi et al., J Eur Acad Dermatol Venereol. 29: 2136-44, 2015; Tan et al., J Cutan Med Surg. 21: 308-315, 2017; Brunner et al., J Allergy Clin Immunol 143: 142-154, 2019).

In still another embodiment, a disease to be diagnosed is seborrheic dermatitis/seborrhoic eczema, and a disease marker to be used is a marker for seborrheic dermatitis/seborrhoic eczema. Examples of markers for seborrheic dermatitis/seborrhoic eczema include IL-2, IFN-$\gamma$, IL-17, and the like (Trznadel-Grodzka et al., Postepy Hig Med Dosw, 66: 843-847, 2012; Wikramanayake et al., Exp Dermatol. 27: 1408-1411, 2018).

In still another embodiment, a disease to be diagnosed is autosensitization dermatitis, and a disease marker to be used is a marker for autosensitization dermatitis. Examples of markers for autosensitization dermatitis include IL-4, IFN$\alpha$, and the like (Tokura et al., J Am Acad Dermatol 57: S22-S25, 2007; Hashimoto et al., J Dermatol 34: 577-582, 2007).

In still another embodiment, a disease to be diagnosed is stasis dermatitis, and a disease marker to be used is a marker for stasis dermatitis. Examples of markers for stasis dermatitis include MMP1, MMP2, MMP13, and the like (Herouy et al., J Dermatol Sci, 25: 198-205, 2001).

In still another embodiment, a disease to be diagnosed is skin pruritus, and a disease marker to be used is a marker for skin pruritus. Examples of markers for skin pruritus include NGFβ, albumin, and the like (Dianis et al., 9th World Congress on Itch. Wroclaw (Poland), 2017/10/15-17 (Abstract)).

In still another embodiment, a disease to be diagnosed is Stevens-Johnson syndrome/mucocutaneous ocular syndrome/EM major and the like, and a disease marker to be used is a marker for Stevens-Johnson syndrome/mucocutaneous ocular syndrome/EM major and the like. Examples of markers for Stevens-Johnson syndrome/mucocutaneous ocular syndrome/EM major include IL-6, IL-10, TNFα, IFNγ, and the like (Ushigome et al., Clin Exp Allergy 48: 1453-1463, 2018.; Ueta et al., BMJ Open Ophthalmol 1: e000073, 2017; Wang et al., J Clin Invest, 128: 985-996, 2018).

In still another embodiment, a disease to be diagnosed is psoriasis, and a disease marker to be used is a marker for psoriasis. Examples of markers for psoriasis include TNFα, IL17, IL22, and the like (Brembilla et al., Front Immunol. 9:1682. 2018).

In still another embodiment, a disease to be diagnosed is alopecia areata, and a disease marker to be used is a marker for alopecia areata. Examples of markers for alopecia areata include IL-15, IL-4, IL-18, and the like (Ebrahim et al., Int J Trichology, 11: 26-30, 2019; Gong et al., Exp Dermatol, doi: 10.1111/exd.13758. [Epub ahead of print], 2018; Moravvej et al., Hum Antibodies, 26: 201-207, 2018; Celik & Ates, J Clin Lab Anal. 32: e22386, 2018).

In still another embodiment, a disease to be diagnosed is male pattern baldness/androgenic alopecia/alopecia prematura, and a disease marker to be used is a marker for male pattern baldness/androgenic alopecia/alopecia prematura. Examples of markers for male pattern baldness/androgenic alopecia/alopecia prematura include SHH, TGFβ, NOTCH1, BMP1, and the like (Minematsu et al., Abstract of the 19th Annual Meeting of Japan Society of Clinical Hair Restoration; Minematsu et al., Abstract of the 20th Annual Meeting of Japan Society of Clinical Hair Restoration).

In still another embodiment, a disease to be diagnosed is cellulitis, and a disease marker to be used is a marker for cellulitis. Examples of markers for cellulitis include TNFα and the like (Mansouri et al., Br J Dermatol, 174: 916-918, 2016).

In still another embodiment, a disease to be diagnosed is folliculitis, and a disease marker to be used is a marker for folliculitis. Examples of markers for folliculitis include TNFα and the like (Werninghaus et al., Clin Exp Dermatol 43: 458-459, 2018).

In still another embodiment, a disease to be diagnosed is tinea/dermatophytosis, and a disease marker to be used is a marker for tinea/dermatophytosis. Examples of markers for tinea/dermatophytosis include keratinase and the like (Takehara et al., the 3rd Annual Meeting of The Society of Nursing Science and Engineering (Abstract); Takehara et al., the 46th Annual Meeting of the Japanese Society for Wound Healing (Abstract)).

In still another embodiment, a disease to be diagnosed is basal cell carcinoma/basal cell epithelioma, and a disease marker to be used is a marker for basal cell carcinoma/basal cell epithelioma. Examples of markers for basal cell carcinoma/basal cell epithelioma include IL-17, IL-22, IL-23, IFNγ, and the like (Pellegrini et al., PLOS One 12: e0183415, 2017).

In still another embodiment, a disease to be diagnosed is squamous cell carcinoma, and a disease marker to be used is a marker for squamous cell carcinoma. Examples of markers for squamous cell carcinoma include CCL5 and the like (Wu et al., Cytokine, 110: 94-103, 2018).

In still another embodiment, a disease to be diagnosed is actinic keratosis/senile keratosis/solar keratosis, and a disease marker to be used is a marker for actinic keratosis/senile keratosis/solar keratosis. Examples of markers for actinic keratosis/senile keratosis/solar keratosis include CD40L and the like (Haimakainen et al., Cancer Invest 35: 143-151, 2017).

In still another embodiment, a disease to be diagnosed is Bowen's disease, and a disease marker to be used is a marker for Bowen's disease. Examples of markers for Bowen's disease include IL-10 and the like (Zhu et al., Gastroenterology Res 10: 65-69, 2017).

In still another embodiment, a disease to be diagnosed is metastatic carcinoma of the skin/malignant wound, and a disease marker to be used is a marker for metastatic carcinoma of the skin/malignant wound. Examples of markers for metastatic carcinoma of the skin/malignant wound include IFNγ and the like (Minematsu et al., Japanese Journal of Pressure Ulcers, 16: 154-158, 2014).

In still another embodiment, a disease to be diagnosed is malignant melanoma, and a disease marker to be used is a marker for malignant melanoma. Examples of markers for malignant melanoma include IL-2, CTLA-4, PD1, and the like (Bridge et al., Front Med: 5:351, 2018).

In still another embodiment, a disease to be diagnosed is laceration (examples of which include skin tear), and a disease marker to be used is a marker for laceration (examples of which include skin tear). Examples of markers for laceration (examples of which include skin tear) include COL4, MMP2, fibronectin, laminin, and the like (Koyano et al., Int Wound J. 13: 189-197. 2016).

In still another embodiment, a disease to be diagnosed is pressure ulcer/pressure injury, and a disease marker to be used is a marker for pressure ulcer/pressure injury. Examples of markers for pressure ulcer/pressure injury include PAI1, HSP90$\alpha$, VEGF-C, IL1$\alpha$, and the like (Nakai et al., Journal of Tissue Viability. 2019. doi: 10.1016/j.jtv.2019.02.002. [Epub ahead of print]; Kimura et al., the 46th Annual Meeting of the Japanese Society for Wound Healing (Abstract)).

In still another embodiment, a disease to be diagnosed is damage of skeletal muscle, and a disease marker to be used is a marker for damage of skeletal muscle. Examples of markers for damage of skeletal muscle include creatine (phospho)kinase and the like (Neya et al., 2018 ASCA International Conference on Applied Strength & Conditioning (Abstract); Neya et al., the 72nd AnnualMeeting of the Japanese Society of Physical Fitness and Sports Medicine (Abstract); Neya et al., the 70th Annual Meeting of the Japanese Society of Physical Fitness and Sports Medicine (Abstract), 16 Sept. 2017).

In still another embodiment, a disease to be diagnosed is malnutrition, and a disease marker to be used is a marker for malnutrition. Examples of markers for malnutrition include albumin, prealbumin, ferritin, and the like (Saarinen & Siimes, Acta Paediatr Scand, 67: 745-751, 1978; Shenkin, Clin Chem, 52:2177-2179, 2006, Gama-Axelsson, Clin J Am Soc Nephrol. 7:1446-1453, 2012).

In still another embodiment, a disease to be diagnosed is hypertonic dehydration, and a disease marker to be used is a marker for hypertonic dehydration. Examples of markers for hypertonic dehydration include taurine and the like (Higashimura et al., Joint Meeting of the 5th Annual Meeting of The Society for Nursing Science and Engineering, the 11th Annual Meeting of the Society of Nursing Practice, and the 7th Annual Meeting of International Lymphoedema Framework Japan (Abstract); Higashimura et al., the 23rd Annual Meeting of Japan Academy of Gerontological Nursing (Abstract); Minematsu et al., the 68th Meeting of The Japan Geriatrics Society - the Kanto Koshinetsu Regional Meeting (Abstract)).

The "reference marker" is annexin A2. Annexin A2 is one of the annexin family proteins, has a molecular weight of 36 kD, and has the C-terminal domain having a core domain that is highly conserved among the annexin family proteins and contains four repeats, each called the annexin repeat, which has an $\alpha$ helical structure containing about 70 amino acid residues. This core domain has a curved structure, and binding sites for $Ca_2^+$ and phospholipid are located on the convex side of the curved structure. In addition, annexin A2, which has no transmembrane domain, exists as a soluble monomer in cytoplasm, and, when expressed in a sufficient amount, annexin A2 binds to S100A10 calcium-binding protein to form a heterotetramer. In addition, an S100A10 calcium-binding protein having a C-terminal lysine binds to the N-terminal domain formed on the concave side of the curved core domain of annexin A2, and thus forms a heterotetramer as a binding site for tissue type plasminogen activator (tPA) and plasminogen. This heterotetramer is characterized by migrating to the cell surface when the Tyr23 of annexin A2 is phosphorylated by a Src-like tyrosine kinase.

In addition, annexin A2 functions, within the cells, as a membrane transport protein, cytoskeleton scaffold protein, or the like, and, on the cell surface, as a plasminogen receptor by binding to an S100A10 calcium-binding protein to form a heterotetramer. The complex of the annexin A2 and the S100A10 calcium-binding protein on the cell surface augments the activation of plasminogen by ten to one hundred times. The plasmin generated on the cell surface by the activation of plasminogen has a property of being not likely to be inhibited by an a2 plasmin inhibitor. In addition, it is known that the activation of plasminogen by the complex of the annexin A2 and the S100A10 calcium-binding protein is associated with the pathologic formation of, for example, inflammatory reaction, thrombosis, autoimmune disease, cancer, or the like.

Annexin A2 is preferably a natural annexin A2 derived from an animal. Examples of "animals" include humans or other mammalian animals (for example, mice, rats, dogs, cats, bovines, horses, pigs, and the like), birds such as domestic fowls and parakeets, and the like. In addition, the word "natural" means that an amino acid sequence of an annexin A2 is naturally-occurring.

It is known that the amino acid sequence of an annexin A2 has a variation (polymorphism) among individuals of the same species. In addition, it is known that some annexin A2s have different amino acid sequences among different species. Annexin A2 encompasses such annexin A2s, the amino acid sequences of which have polymorphisms or are different among different species, and the like.

It is known that the annexin A2 of a human has four types of mRNA splicing variants. The cDNAs corresponding to the four types of mRNA splicing variants are registered as Accession Nos. NM_001002857.1 (SEQ ID NO: 1), NM_001002858.2 (SEQ ID NO: 3), NM_001136015.2 (SEQ ID NO: 5), and NM_004039.2 (SEQ ID NO: 7) in the database of NCBI. In addition, the proteins corresponding to these cDNAs are registered as Accession Nos. NP_001002857.1 (SEQ ID NO: 2), NP_001002858.1 (SEQ ID NO: 4), NP_00129487.1 (SEQ ID NO: 6), and NP004030.1 (SEQ ID NO: 8) in the database of NCBI.

The cDNA of the annexin A2 of a rat is registered as Accession No. NM_019905.1 (SEQ ID NO: 9) in the database of NCBI, and the corresponding protein is registered as Accession No. NP_063970.1 (SEQ ID NO: 10).

Examples of an annexin A2 to be used as a reference marker include: a protein composed of one amino acid sequence selected from the group consisting of SEQ ID NOs: 2, 4, 6, 8, and 10; and a protein which is composed of an amino acid sequence having an identity of 80% or more, preferably 85% or more, more preferably 90% or more, still more preferably 95% or more, still more preferably 98% or more, still more preferably 99% or more, to one amino acid sequence selected from the group consisting of SEQ ID NOs: 2, 4, 6, 8 and 10 and which can specifically bind to an anti-annexin A2 monoclonal antibody.

The "diagnosis of a disease" encompasses a doctor's determining the health condition of a subject based on information related to the health condition of the subject, and/or a doctor's selecting a method for preventing or treating a disease in a subject based on information related to the health condition of the subject, followed by determining the necessity to perform the method. The health condition encompasses: a subject's current health condition (for example, the presence or absence of a disease, the severity of a disease, the likelihood of contracting a disease, and the like), a subject's future health condition (for example, the prognosis of a disease and the like), and the like.

"Assisting diagnosis of a disease" encompasses assisting a doctor in determining the health condition of a subject based on information related to a disease in the subject, and/or assisting a doctor in selecting a method for preventing or treating a disease in a subject based on information related to the disease in the subject, followed by determining the necessity to perform the method. The health condition encompasses: a subject's current health condition (for example, the presence or absence of a disease, the severity of a disease, the likelihood of contracting a disease, and the like), a subject's future health condition (for example, the prognosis of a disease and the like), and the like. The act of assisting diagnosis of a disease is the act of providing information useful for diagnosis of the disease, may be a medical act or a non-medical act, and is usually a non-medical act. A doctor's diagnosis can be performed based on not only information provided by the act of assisting diagnosis of a disease but also one or more kinds of other information. In addition, a doctor's diagnosis can involve the doctor's experience, know-how, and the like. For this reason, the expression "assisting" is used in the present invention. Accordingly, the expression "assisting" does not exclude performing diagnosis of a disease in a subject based on information provided by the act of assisting diagnosis of a disease. In other words, the expression "assisting" as used herein also encompasses performing diagnosis of a disease in a subject based on information provided by the act of assisting diagnosis of a disease.

<First Aspect>

A first aspect of the present invention relates to a method of assisting diagnosis of a disease in a subject using a disease marker and a reference marker.

The method according to the first aspect of the present invention includes the following steps. Steps (a) to (d) are sequentially carried out.

(a) A step of providing a sheet capable of generating an attractive force due to electrostatic interaction between the sheet and each of the disease and reference markers;
(b) A step of applying the sheet to a skin surface of the subject, followed by detaching the sheet from the skin surface;
(c) A step of measuring an amount of each of the disease and reference markers that are attached to the sheet; and
(d) A step of acquiring information on the disease in the subject by using a value obtained by correcting the amount of the disease marker measured in step (c) with the amount of the reference marker measured in step (c).

Below, each step will be described.

Step (a)

Step (a) is a step of providing a sheet capable of generating an attractive force due to electrostatic interaction between the sheet and each of the disease and reference markers.

The sheet provided in step (a) is not limited to any particular sheet as long as the sheet is capable of generating an attractive force due to electrostatic interaction between the sheet and each of the disease and reference markers. The electrostatic interaction encompasses interionic interaction, dipole-dipole interaction, ion-dipole interaction, and the like. The interionic interaction can occur between the ionized functional group of the disease marker and/or the reference marker in interstitial fluid of the subject and the ionized functional group of the sheet. The dipole-dipole interaction can occur between the polarized functional group (the functional group having a dipole moment) of the disease marker and/or the reference marker in interstitial fluid of the subject and the polarized functional group (the functional group having a dipole moment) of the sheet. The ion-dipole interaction can occur between the polarized functional group (the functional group having a dipole moment) of the disease marker and/or the reference marker in interstitial fluid of the subject and the ionized functional group of the sheet, or between the ionized functional group of the disease marker and/or the reference marker in interstitial fluid of the subject and the polarized functional group (the functional group having a dipole moment) of the sheet.

Examples of the sheet provided in step (a) include a sheet having a polar functional group on the surface of the sheet, and the like. The sheet having a polar functional group can generate an attractive force due to electrostatic interaction between the positive charge of the polar functional group and the negative charge of a disease marker and/or a reference marker, and/or an attractive force due to electrostatic interaction between the negative charge of the polar functional group and the positive charge of a disease marker and/or a reference marker.

The polar functional group is a functional group having polarity. The polarity of a functional group can be generated by the polarization of the functional group. A functional group is polarized by, for example: a difference in electronegativity between the atoms contained in the functional group; a difference in electronegativity between the atom contained in the functional group and the atom (for example, the carbon atom) bound to the functional group; a combination thereof; and the like. The polar functional group may be a nonionic functional group or an ionic functional group.

The nonionic functional group is a functional group that is not ionized in an aqueous medium having a neutral or approximately neutral pH (pH 6.5 to 7.5) (for example, water, physiological saline, phosphate buffered saline, or the like), and examples of nonionic functional groups include nitro groups and the like.

The ionic functional group is a functional group that is ionized and positively or negatively charged in an aqueous medium having a neutral or approximately neutral pH (pH 6.5 to 7.5) (for example, water, physiological saline, phosphate buffered saline, or the like), and examples of ionic functional groups include cationic functional groups, anionic functional groups, and the like.

Examples of cationic functional groups include a primary amino group ($-NH_2$), a secondary amino group ($-NHR$), a tertiary amino group ($-NR_2$), a quaternary ammonium group ($-NR_3^+$), a quaternary phosphonium group ($-PR_3^+$), and the like. R independently represents an organic group such as an optionally substituted alkyl group, optionally substituted alkoxy group, or optionally substituted aryl group. Among the cationic functional groups, quaternary ammonium groups and quaternary phosphonium groups can be positively charged independent of the pH of an aqueous medium. The cationic functional group may bind to an anion such as a halogen anion, sulfate anion, sulfonate anion, phosphate anion, or carboxylate anion to form a salt.

Examples of anionic functional groups include hydroxyl groups, carboxylic groups, sulfo groups, sulfate groups, phosphate groups, silanol groups ($-SiRR'OH$), and the like. Examples of hydroxyl groups include phenolic hydroxyl groups ($-Ar-OH$). R and R' independently represent an organic group such as an optionally substituted alkyl group, or optionally substituted alkoxy group. The anionic functional group may bind to an alkali metal ion such as a sodium ion or potassium ion to form a salt.

Examples of sheets having a polar functional group on the respective surfaces of the sheets include nitrocellulose membranes, nylon membranes, and the like. Examples of nitrocellulose membranes and nylon membranes that can be used include commercially available membranes for blotting of proteins, nucleic acids, and the like. The nitrocellulose membrane may be composed of nitrocellulose alone, or may have a substrate (for example, a substrate made of a polyester resin) for supporting nitrocellulose. The nitrocellulose membrane and nylon membrane may be electrically neutral as the whole sheet, or may be positively or negatively charged as the whole sheet.

The nitrocellulose membrane is preferably used as a sheet having a polar functional group on the surface of the sheet. The nitrocellulose membrane can generate an attractive force due to electrostatic interaction between the positive charge of the nitro group and the negative charge of a disease marker and/or a reference marker, and an attractive force due to electrostatic interaction between the negative charge of the nitro group and the positive charge of a disease marker and/or a reference marker.

The nitration ratio of the nitrocellulose membrane is preferably 30% or more, more preferably 35% or more. The "nitration ratio of a nitrocellulose membrane" is calculated based on the amount of $NO_2^-$ and the amount of $NO_3^-$ in accordance with the method described in Analytica Chimica Acta 685 (2011) 196-203, wherein the $NO_2^-$ and $NO_3^-$ are generated by hydrolyzing the nitro group with alkali, and quantitated by ion chromatography. Specifically, this is as below-mentioned. A nitrocellulose fraction is solvent-extracted from a nitrocellulose membrane in question. To the nitrocellulose fraction, a 20 g/L NaOH solution is added, and the resulting mixture is heated at 150°C for 30 minutes to hydrolyze the nitro group. The hydrolyzed dry residue is dissolved in distilled water to prepare a sample solution. By ion chromatography, $NO_2^-$ and $NO_3^-$ in the sample solution are quantitated. Based on the $NO_2^-$ amount and $NO_3^-$ amount, the nitro group content (mmol/g) of the sample solution is calculated. Completely nitrated nitrocellulose is hydrolyzed in the same manner as above-mentioned, and the nitro group content (mmol/g) of the completely nitrated nitrocellulose is calculated based on the $NO_2^-$ amount and $NO_3^-$ amount quantitated by ion chromatography. Based on the following equation, the nitration ratio (%) of the nitrocellulose membrane in question is calculated.

$$\text{Nitration ratio (\%)} = (\text{nitro group content of sample solution})/(\text{nitro group content of completely nitrated nitrocellulose}) \times 100$$

In this regard, the nitro group content of completely nitrated nitrocellulose is assumed to be 9 mmol/g.

Other examples of sheets having a polar functional group on the respective surfaces of the sheets include: a sheet having a surface that is or can be positively charged, and being capable of generating an attractive force due to electrostatic interaction with the negative charge of a disease marker and/or a reference marker; a sheet having a surface that is or can be negatively charged, and being capable of generating an attractive force due to electrostatic interaction with the positive charge of a disease marker and/or a reference marker; and the like. Examples of such sheets include anion exchange membranes, cation exchange membranes, and the like. Commercially available anion exchange membranes and cation exchange membranes can be used. The description of the cationic functional group of a surface of the anion exchange membrane and the anionic functional group of a surface of the cation exchange membrane is the same as mentioned above, and thus, omitted here.

The anion exchange membrane is allowed to have a positively charged surface by the ionization of the cationic functional group, resulting in a sheet capable of generating an attractive force due to electrostatic interaction with the negative charge of a disease marker and/or a reference marker. The ionization of the cationic group can be caused by, for example, wetting the anion exchange membrane with an aqueous medium. The anion exchange membrane may have an anionic group as long as the membrane can generate an attractive force due to electrostatic interaction with the negative charge of a disease marker and/or a reference marker.

The cation exchange membrane is allowed to have a negatively charged surface by the ionization of the anionic group, resulting in a sheet capable of generating an attractive force due to electrostatic interaction with the positive charge of a disease marker and/or a reference marker. For example, when the disease marker is taurine, a sheet obtained by combining a nitrocellulose membrane and a cation exchange membrane is used. The ionization of the anionic group can be caused by, for example, wetting the cation exchange membrane with an aqueous medium. The cation exchange membrane may have a cationic group as long as the membrane can generate an attractive force due to electrostatic interaction with the positive charge of a disease marker and/or a reference marker.

Besides these, a sheet obtained by introducing a cationic group into a suitable substrate (for example, a water-insoluble substrate) may be used as a sheet having a surface that is or can be positively charged, and being capable of generating an attractive force due to electrostatic interaction with the negative charge of a disease marker and/or a reference marker. Examples of substrates include nitrocellulose membranes, nylon membranes, polyvinylidene fluoride membranes, cellulose ester membranes such as of cellulose acetate, polyester membranes such as of polyethylene terephthalate, polyolefinic resin membranes such as of polyethylene, polypropylene, and polystyrene, and the like. The substrate may have an anionic group as well as a cationic group introduced thereinto as long as the obtained sheet can generate an attractive force due to electrostatic interaction with the negative charge of a disease marker and/or a reference marker.

Besides these, a sheet obtained by introducing an anionic group into a suitable substrate (for example, a water-insoluble substrate) may be used as a sheet having a surface that is or can be negatively charged, and being capable of generating an attractive force due to electrostatic interaction with the positive charge of a disease marker and/or a reference marker. Examples of substrates include nitrocellulose membranes, nylon membranes, polyvinylidene fluoride membranes, cellulose ester membranes such as of cellulose acetate, polyester membranes such as of polyethylene terephthalate, polyolefinic resin membranes such as of polyethylene, polypropylene, and polystyrene, and the like. The substrate may have a cationic group as well as an anionic group introduced thereinto as long as the obtained sheet can generate an attractive force due to electrostatic interaction with the positive charge of a disease marker and/or a reference marker.

Without any particular limitation, the shape, size, and the like of the sheet can suitably be adjusted in accordance with the shape, size, and the like of a skin surface to which the sheet is applied. The sheet may be a combination of a plurality of sheet members of the same kind or different kinds, and may be, for example, a plurality of sheet members disposed on the same plane, a laminate of a plurality of sheet members, or the like. For example, what can be used for a disease marker that is taurine is a sheet obtained by combining a nitrocellulose membrane and an anion exchange membrane, specifically a sheet obtained by disposing and fixing, on an adhesive substrate, an outer frame which is a nitrocellulose membrane, and an anion exchange membrane inside the outer frame which is a nitrocellulose membrane.

The sheet is preferably flexible. The sheet that is flexible enables the shape of the sheet to be deformed in conformance with the shape of a skin surface, thus making it easy to bring the sheet into contact with the skin surface.

The sheet may have liquid permeability. For example, the sheet may have liquid-permeable pores (for example, penetrating holes having a diameter of 0.2 to 0.45 $\mu$m).

Step (b)

Step (b) is a step of applying the sheet provided in step (a) to a skin surface of the subject, followed by detaching the sheet from the skin surface.

When the sheet is applied to a skin surface of the subject, an attractive force due to electrostatic interaction between the sheet and each of the disease and reference markers in interstitial fluid existing in a layer located below the skin surface (for example, one or more layers selected from the stratum spinosum and stratum basale of the epidermis, the dermis, the subcutaneous fat, the skeletal muscle, and the like) is generated, and thus the disease and reference markers in interstitial fluid of the subject are attracted to the sheet by passing through the epidermis of the subject, and are attached to the surface of the sheet. In other words, the disease and reference markers in interstitial fluid of the subject are noninvasively collected. Other than the electrostatic interaction, a hydrophobic interaction and the like may be responsible for the binding between the sheet and each of the disease and reference markers attached to the sheet surface.

The layer located below the skin surface of the subject encompasses: a layer serving the skin barrier function (the sebum barrier of the skin surface, the stratum corneum and stratum granulosum of the epidermis, and the like); and a layer located below the layer serving the skin barrier function (for example, one or more layers selected from the stratum spinosum and stratum basale of the epidermis, the dermis, the subcutaneous fat, the skeletal muscle, and the like). In the layer serving the skin barrier function, no interstitial fluid exists, but a substance that is the same as a disease marker can exist. Even if a substance existing in the layer serving the skin barrier function is the same as a disease marker, the substance does not reflect the health condition of the subject, and does not serve as an index for a disease. The disease markers attached to the sheet surface can contain a substance that is the same as the disease marker and that exists in the layer serving the skin barrier function, in addition to the disease marker existing in interstitial fluid of a layer located below the layer serving the skin barrier function. However, the amount of the substance that is the same as the disease marker and that exists in the layer serving the skin barrier function is very small, and thus, even if in addition to the disease marker, such a substance is attached to the sheet surface, this will not be particularly problematic.

When the sheet is detached from the skin surface, the disease and reference markers attached to the surface of the sheet are detached, together with the sheet, from the skin surface. To the sheet detached from the skin surface, some substances that are among substances existing in the skin surface and that have affinity with the sheet surface (for example, proteins, lipids, and the like other than the disease marker and the reference marker) may be attached.

The time taken to contact the sheet with the skin surface is not limited to any particular value as long as the disease and reference markers in interstitial fluid of the subject can be attached to the sheet surface. The contact time can suitably be adjusted in accordance with, for example, the magnitude of an attractive force due to electrostatic interaction generated between the sheet and each of the reference and disease markers in interstitial fluid of the subject, and usually one minute or more, preferably five minutes or more, more preferably ten minutes or more. The upper limit of the contact time is not limited to any particular value, and is usually 20 minutes, preferably 15 minutes.

In step (b), it is preferable that one or both of the sheet and the skin surface is/are wetted with an aqueous medium, and that the sheet is then applied to the skin surface. This makes it possible to remove air existing between the sheet and the skin surface, and consequently makes it possible to prevent the electrostatic interaction from being attenuated by the air existing between the sheet and the skin surface. Examples of aqueous media that can be used include aqueous media having a neutral or approximately neutral pH (pH 6.5 to 7.5) (for example, water, physiological saline, phosphate buffered saline, and the like).

Step (c)

Step (c) is a step of measuring an amount of each of the disease and reference markers that are attached to the sheet.

When the disease marker is a protein, the amount of each of the disease and reference markers can be measured, for example, using a labeled antibody that recognizes the disease marker and a labeled antibody that recognizes the reference marker. As an antibody that recognizes the disease or reference marker, commercially available monoclonal antibodies or polyclonal antibodies may be used, or an antibody or antiserum collected through immunizing an animal such as a rabbit or a mouse with each of the disease and reference markers may be used. Examples of labeled substances include fluorescent dyes (for example, fluoresceins, rhodamines, coumarins, pyrenes, cyanines, and the like), radioactive substances (for example, $^{13}C$, $^{3}H$, and the like), enzymes (for example, alkaline phosphatase, peroxidase, and the like), coloring particles (for example, metal colloid particles, coloring latexes, and the like), and the like. Examples of methods of measuring the amount of each of the disease and reference markers using a labeled antibody include enzyme immunoassay, radioimmunoassay, Western blotting, and the like. When the disease marker is a protein, the amount of the protein can be measured, for example, by allowing a labeled antibody that recognizes the protein to be bound to the protein attached to the sheet, measuring the amount of the bound labeled antibody, and applying the measured value to a calibration curve. Such a calibration curve to be used may be prepared preliminarily, or prepared every time a measurement is made.

When the disease marker is an osmolyte, the amount of the disease marker can be measured using a substance that recognizes the disease marker, examples of such substances including fluorescence-labeled dyes, labeled antibodies, and the like. For example, when the disease marker is taurine, the amount of the taurine can be measured, for example, by allowing the taurine attached to the sheet to be stained with ninhydrin as a fluorescence-labeled dye, measuring the staining intensity to determine the absorbance of light at a wavelength of 450 nm, and applying the determined value to a calibration curve. Such a calibration curve to be used may be prepared preliminarily, or prepared every time a measurement is made.

Step (d)

Step (d) is a step of acquiring information on the disease in the subject by using a value obtained by correcting the amount of the disease marker measured in step (c) with the amount of the reference marker measured in step (c).

The disease marker amount is corrected by obtaining a value (a corrected value) obtained by dividing the value of the disease marker amount measured in step (c) by the value of the reference marker amount measured in step (c). Based on the obtained corrected value, information on the health condition of the subject is acquired.

Information on a disease means information on whether a subject is suffering from the disease, information on whether a subject is suspected of being suffering from the disease, and the like. In addition, information on a disease includes a time course of a plurality of information on the disease obtained over time.

Information on the health condition of a subject can be obtained by comparing the corrected value of the disease marker amount of the subject with a preliminarily set reference value in accordance with, for example, a known method such as a simple comparative method, statistical test, or the like.

The reference value is preferably determined based on the disease marker amount measured in the same manner as steps (a) to (c), except that the measure is carried out with respect to a healthy individual(s) belonging to the same species as the subject. In determining the reference value based on the disease marker amount of the individuals, the reference value can be determined, for example, based on an average value, a median value or the like of the disease marker amounts of the individuals. In determining the reference value, the healthy individual belonging to the same species as the subject is preferably an individual having the same gender as the subject or an individual having an age closer to the subject's age, more preferably an individual having the same gender as the subject and having an age closer to the subject's age.

The health condition of the subject can be determined based on, for example: the corrected value obtained by using the disease marker amount and the reference marker amount measured at a point of time; a difference among the corrected values obtained by using the disease marker amounts and the reference marker amounts measured by carrying out steps (a) to (c) at a plurality of points of time; and the like.

For example, in cases where a determination is made on whether a subject is suffering from a disease, the fact that the corrected value of the disease marker amount is equal to or greater than the reference value makes it possible to determine that the subject is suffering from or suspected of being suffering from the disease that can be determined using the disease marker as an index. On the other hand, the fact that the corrected value of the disease marker amount is smaller than the reference value makes it possible to determine that the subject is not suffering from the disease that can be determined using the disease marker as an index.

For example, in cases where a determination is made on the severity of a disease, the fact that the corrected value of the disease marker amount is equal to or greater than the reference value, and significantly different from the reference value makes it possible to determine that the subject is severely suffering from the disease that can be determined using the disease marker as an index. On the other hand, the fact that the corrected value of the disease marker amount is equal to or greater than the reference value, and not very different from the reference value makes it possible to determine that the subject is mildly suffering from the disease that can be determined using the disease marker as an index.

For example, in cases where a determination is made on the likelihood of contracting a disease, the fact that the corrected value of the disease marker amount is equal to or smaller than the reference value, and significantly different from the reference value makes it possible to determine that the subject is not likely to contract the disease that can be determined using the disease marker as an index. On the other hand, the fact that the corrected value of the disease marker amount is equal to or smaller than the reference value, and not very different from the reference value makes it possible to determine that the subject is likey to contract the disease that can be determined using the disease marker as an index.

For example, the fact that the corrected value of the disease marker amount exhibits a tendency to increase makes it possible to determine that the subject has a tendency to more easily contract the disease that can be determined using the disease marker as an index, or that the disease has a tendency to become more severe. On the other hand, the fact that the corrected value of the disease marker amount exhibits a tendency to decrease makes it possible to determine that the subject has a tendency to less easily contract the disease that can be determined using the disease marker as an index, or that the disease has a tendency to become improved.

The method according to the first aspect of the present invention makes it possible to obtain as information useful for assisting diagnosis of a disease of a subject, information accurately reflecting the actual health condition of the subject, regardless of variation in the skin barrier function of the subject, by correcting variation in the amount of a collected disease marker, which is caused by variation in the skin barrier function of the subject.

A doctor's diagnosis can be performed based on not only the corrected value obtained in step (d) and information acquired based on the corrected value but also one or more kinds of other information. In addition, a doctor's diagnosis can involve the doctor's experience, know-how, and the like. For this reason, the expression "assisting" is used in the present invention. Accordingly, the expression "assisting" does not exclude performing diagnosis of a disease in a subject based on the corrected value obtained in step (d) and information acquired based on the corrected value. In other words, the present invention encompasses performing diagnosis of a disease in a subject based on the corrected value obtained in step (d) and information acquired based on the corrected value.

<Second Aspect>

A second aspect of the present invention relates to a method of collecting a disease marker and a reference marker derived from interstitial fluid of a subject to assist diagnosis of a disease in the subject.

The method according to the second aspect of the present invention includes the following steps, wherein the reference marker is annexin A2. Steps (a) and (b) are sequentially carried out.

(a) A step of providing a sheet capable of generating an attractive force due to electrostatic interaction between the sheet and each of the disease and reference markers; and
(b) A step of applying the sheet to a skin surface of the subject, followed by detaching the sheet from the skin surface.

Steps (a) and (b) in the method according to the second aspect of the present invention are the same as the steps (a) and (b) in the method according to the first aspect of the present invention, and thus, the description is omitted.

The method according to the second aspect of the present invention makes it possible to transcutaneously and noninvasively collect disease and reference markers derived from interstitial fluid of the subject to assist diagnosis of a disease in the subject.

<Third Aspect>

A third aspect of the present invention relates to a kit for assisting diagnosis of a disease in a subject.

The kit according to the third aspect of the present invention includes a sheet capable of generating an attractive force due to electrostatic interaction between the sheet and each of the disease and reference markers, wherein the reference marker is annexin A2.

The sheet in the kit according to the third aspect of the present invention is the same as the sheet provided in step (a) in the method according to the first aspect of the present invention, and thus, the description is omitted.

It is preferable that the kit according to the third aspect of the present invention further includes a first reagent for detecting the disease marker and a second reagent for detecting the reference marker. The first reagent and the second reagent are preferably a labeled antibody that recognizes the disease marker and a labeled antibody that recognizes reference marker, respectively, which are used in step (c) in the method according to the first aspect of the present invention.

Specifically, when the disease marker is a protein, the first reagent is preferably a labeled antibody that recognizes the protein.

In addition, when the disease marker is an osmolyte, the first reagent is preferably a fluorescence-labeled dye, a labeled antibody, or the like that recognizes the osmolyte. More specifically, when the disease marker is taurine, the first reagent is preferably an annexin that binds to taurine.

In addition, the second reagent is preferably a labeled antibody that recognizes a disease marker.

The kit according to the third aspect of the present invention can be used to carry out the method according to the first aspect of the present invention. Accordingly, a kit according to the third aspect of the present invention makes it possible that, in order to assist diagnosis of a disease in a subject, information accurately reflecting the actual health condition of the subject is obtained with respect to the disease in the subject, regardless of variation in the skin barrier function of the subject, by correcting variation in the amount of a collected disease marker, which is caused by variation in the skin barrier function of the subject.

<Fourth Aspect>

A fourth aspect of the present invention relates to a kit for collecting a disease marker and a reference marker derived from interstitial fluid of a subject to assist diagnosis of a disease in the subject.

The kit according to the fourth aspect of the present invention includes a sheet capable of generating an attractive force due to electrostatic interaction between the sheet and each of the disease and reference markers, wherein the reference marker is annexin A2.

The sheet in the kit according to the fourth aspect of the present invention is the same as the sheet provided in step (a) in the method according to the first aspect of the present invention, and thus, the description is omitted.

It is preferable that the kit according to the fourth aspect of the present invention further includes a first reagent for detecting the disease marker and a second reagent for detecting the reference marker. The first reagent and second reagent in the kit according to the fourth aspect of the present invention are the same as the first reagent and second reagent respectively included in the kit according to the third aspect of the present invention, and thus, the description is omitted.

The kit according to the fourth aspect of the present invention can be used to carry out the method according to the second aspect of the present invention. Accordingly, the kit according to the fourth aspect of the present invention makes it possible to transcutaneously and noninvasively collect disease and reference markers derived from interstitial fluid of a subject to assist diagnosis of a disease in the subject.

EXAMPLES

1. Verification of validity of model animals

The following four types of rats (SD, male, five rats per type) were provided as candidates of model animals for verification of the effects of the present invention.

Young rats: ten-week-old rats
Aged rats: six-month-old rats

Dry skin rats: the back was shaved, and the shaved site was treated by applying thereto an acetone-ethanol solution mixture once per day in three days.

Ultraviolet-irradiated rats: the back was shaved, and the shaved site was irradiated with ultraviolet light (UV-B) for one minute.

In accordance with the following procedures, whether the four types of rats are each valid as a model animal was verified.

The total RNA was extracted from a tissue sample collected from each of the four types of rats, and the expression levels of the sirtuin 1 gene (Sirt 1) and sirtuin 2 gene (Sirt 2), which are known as being expressed increasingly with aging, were measured based on the extracted total RNA.

In addition, the expression level of an interleukin-lb gene (Il-1b) known as an inflammatory cytokine was measured.

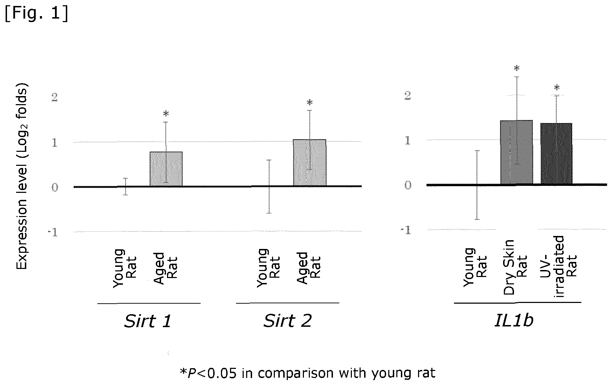

As shown in FIG. 1, the Sirt 1 and Sirt 2 genes both exhibited a significantly higher expression level in the aged rats than in the young rats.

Also as shown in FIG. 1, the Il-1b gene exhibited a significantly higher expression level in the dry skin rats and ultraviolet-irradiated rats than in the young rats.

These results have revealed that the above-mentioned four types of rats can be used as model animals reflecting the actual skin condition accurately.

2. Collection of Samples

In accordance with the following procedures, tissue samples and skin blotting samples were collected from the above-mentioned four types of rats (five rats per type), the validity of which as model animals was verified. From the dry skin rats, samples were collected two days after the final application of the acetone-ethanol solution mixture. When the samples were collected, the age was 10 weeks. In addition, samples were collected from the ultraviolet-irradiated rats two days after the ultraviolet-irradiation. When the samples were collected, the age was 10 weeks.

Collection of tissue samples: after the skin blotting samples were collected, skins and subcutaneous adipose tissues were collected from the four types of rats, using dissecting scissors, at the same site as the rats were subjected to skin blotting. The collected tissues were divided into two: one was used to extract the total RNA, and the other to produce fixed tissues.

Collection of skin blotting samples: one drop of physiological saline was applied dropwise to a 1 cm $\times$ 0.5 cm supported nitrocellulose membrane (manufactured by Bio-Rad Laboratories, Inc.), and the resulting membrane was applied to the back skin for ten minutes, followed by detaching the membrane from the back skin. The detached nitrocellulose membrane was dried at 4°C.

3. Search for reference marker

A reference marker to be used for correcting a disease marker in the present invention was searched for by the following procedures.

With respect to the 11 types of genes encoding the secretory proteins listed in Table 1 among the 566 types of house-keeping genes listed in Eisenberg E & Levanon EY., Trends Genet. 2003;19(7): 362-5, a real-time PCR was carried out on a cDNA obtained by reverse transcription from the total RNA collected from the tissue sample of each of the four types of rats. A primer-probe solution mixture, TaqMan Gene Expression Assay (manufactured by Thermo Fisher Scientific Inc.), specific to each cDNA was used. In addition, an 18S ribosome RNA (Eukaryotic 18S rRNA Endogenous Control, manufactured by Thermo Fisher Scientific Inc.) was used as a positive control. With respect to the four types of rats, the results of the real-time RT-PCR of the 11 types of genes (Ct values) are shown in Table 1.

[Table 1]

| Candidate Gene | Young Rat (average ± standard deviation) | Aged Rat (average ± standard deviation) | Dry Skin Rat (average ± standard deviation) | UV-irradiated Rat (average ± standard deviation) |
|---|---|---|---|---|
| 18S rRNA | 15.57 ± 1.548 | 15.91 ± 1.251 | 15.44 ± 1.637 | 15.12 ± 0.871 |
| Csf1 | 35.64 ± 2.370 | 35.76 ± 1.424 | 35.59 ± 1.413 | 35.24 ± 1.228 |
| Mrc2 | 36.54 ± 2.966 | 35.82 ± 1.444 | 35.37 ± 1.712 | 35.22 ± 1.123 |
| Col6a1 | 33.04 ± 2.339 | 33.70 ± 1.364 | 32.82 ± 1.546 | 32.17 ± 0.903 |
| Lyz2 | 35.10 ± 2.246 | 34.76 ± 1.178 | 34.61 ± 1.685 | 33.46 ± 1.208 |
| B2m | 29.11 ± 2.109 | 28.61 ± 1.357 | 28.70 ± 1.861 | 28.28 ± 1.034 |
| Anxa2 | 31.08 ± 1.420 | 31.10 ± 1.461 | 30.66 ± 1.835 | 30.25 ± 1.187 |
| Chit1 | 40.02 ± 1.248 | 39.79 ± 1.400 | 38.24 ± 2.428 | 37.58 ± 1.087 |
| Gpi | 30.06 ± 1.892 | 30.62 ± 1.408 | 30.21 ± 1.404 | 29.81 ± 1.142 |
| Efna3 | 33.30 ± 1.908 | 32.86 ± 2.366 | 32.41 ± 1.944 | 32.15 ± 1.316 |
| Vegfb | 32.88 ± 1.538 | 32.90 ± 1.214 | 33.07 ± 1.248 | 32.38 ± 1.077 |
| Cstb | 32.60 ± 1.822 | 31.88 ± 1.886 | 31.24 ± 1.928 | 30.98 ± 1.050 |

The reference marker preferably exists in a given or larger amount in the skin tissue, and thus, a verification test was further carried out on the seven types of genes: B2m, Gpi, Anxa 2, Cstb, Efna 3, Vegfb, and Col6a1, each of which had a Ct value of less than 35 in any of the four types of rats.

All the 20 rats of the four types were analyzed for correlation between the expression amount of each of the seven types of genes and the expression amount of the positive control 18S rRNA. The analysis results are shown in Table 2.

[Table 2]

| Candidate Gene | Correlation with 18S rRNA | |
|---|---|---|
| | r | P |
| 18S rRNA | - | - |
| Col6a1 | 0.936 | <0.001 |
| B2m | 0.894 | <0.001 |
| Anxa2 | 0.902 | <0.001 |
| Gpi | 0.869 | <0.001 |
| Efna3 | 0.853 | <0.001 |
| Vegfb | 0.865 | <0.001 |
| Cstb | 0.837 | <0.001 |

In any cell of the skin tissue, 18S rRNA exhibited a stable expression amount, and thus, the higher the correlation with the expression amount of 18S rRNA is, the more stable the expression amount of each of the seven types of genes can be said to be in the cell of the skin tissue.

The results in Table 2 revealed that the expression amounts of the two types of genes, Col6a1 and Anxa 2, among the seven types of genes exhibited a particularly high correlation (a correlation coefficient $r \geq 0.9$) with the expression amount of 18S rRNA, and thus, a verification test was further carried out on these two types of genes.

With respect to each of the four types of rats, a correction was made by dividing the Ct values of the two types of genes by the total RNA amount of the tissue sample of each rat. The corrected Ct values and the results of analysis of variance (p values) are shown in Table 3.

[Table 3]

| Candidate Gene | Young Rat (average ± standard deviation) | Aged Rat (average ± standard deviation) | Dry Skin Rat (average ± standard deviation) | UV-irradiated Rat (average ± standard deviation) | P value |
|---|---|---|---|---|---|
| 18S rRNA | 18.32 ± 0.356 | 19.29 ± 0.883 | 19.02 ± 0.974 | 18.27 ± 0.708 | 0.261 |
| Col6a1 | 35.80 ± 0.779 | 37.08 ± 0.825 | 36.40 ± 0.779 | 35.32 ± 0.628 | 0.024 |
| Anxa2 | 33.84 ± 0.331 | 34.47 ± 0.531 | 34.23 ± 1.118 | 33.40 ± 0.864 | 0.353 |

The results in Table 3 have revealed that Anxa 2 did not exhibit a significant difference in the corrected Ct value among the four types of rats ($p \geq 0.05$). A reference marker preferably exists in the skin tissue independent of the skin condition, and thus, annexin A2 was found to be preferable as a reference marker.

Furthermore, Anxa 2 and 18S rRNA which was a positive control were measured for the expression amount in each of the four types of rats, and the coefficient of variation (CV value) was calculated. The results are shown in Table 4.

[Table 4]

| Candidate Gene | CV |
|---|---|
| 18S rRNA | 0.045 |
| Anxa2 | 0.024 |

According to the results in Table 4, Anxa 2 exhibited a smaller CV value than 18S rRNA. The expression amount of a reference marker is preferably less different among individuals independent of age, health condition, and the like, and thus, the results in Table 4 have also revealed that annexin A2 is preferable as a reference marker.

4. Distribution of annexin A2 (Anxa 2) in skin

The tissue samples from all the rats were immunostained in accordance with the following procedures.

(1) Section production: a tissue sample was fixed in 4% paraformaldehyde and embedded in paraffin to produce a section having a thickness of approximately 3 μm.

(2) Blocking: the section obtained in (1) was deparaffinized and the resulting section was immersed in a blocking agent, Blocking One (manufactured by Nacalai Tesque, Inc.), and allowed to undergo blocking for one hour.

(3) Primary antibody reaction: the section obtained in (2) was immersed in a diluent of an anti-annexin A2 antibody (manufactured by Cell Signaling Technology, Inc.) (at a dilution ratio of 1:200), and allowed to react for one hour.

(4) Washing: the section obtained in (3) was washed.

(5) Secondary antibody reaction: the section obtained in (4) was immersed in a diluent of a horseradish peroxidase (HRP)-labeled antirabbit IgG antibody (manufactured by Jackson ImmunoResearch Inc.) (at a dilution ratio of 1:1000), and allowed to react for one hour.

(6) Washing: the section obtained in (5) was washed.

(7) Staining: the section obtained in (6) was colored with a chromogenic substrate 3,3'-diaminobenzidine (DAB, manufactured by Wako Pure Chemical Industries, Ltd.), and then, stained with hematoxylin.

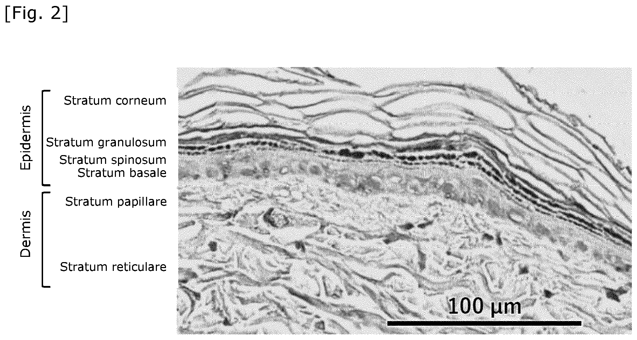

The stained section of a young rat is shown in FIG. 2.

A reference marker is used to correct variation in the amount of a disease marker, which is caused by variation in the skin barrier function, and when being collected, a reference marker needs to pass through the stratum corneum and stratum granulosum of the epidermis, which serve the skin barrier function. Thus, the reference marker needs to exist in one or more layers selected from the stratum spinosum, stratum basale, dermis, subcutaneous fat, skeletal muscle, and the like that are located below (inside) the stratum corneum and stratum granulosum of the epidermis. FIG. 2 shows that annexin A2 abounds in the stratum spinosum and stratum basale of the epidermis of the skin. Accordingly, these results have also revealed that annexin A2 is preferable as a reference marker.

## 5. Staining of skin blotting sample

The skin blotting sample collected from each of the four types of rats was stained in accordance with the following procedures (1) to (6) using a vacuum driven staining system, SNAP i.d. 2 (manufactured by Merck Millipore).

(1) Blocking: the collected skin blotting sample was immersed in a blocking agent Blocking One (manufactured by Nacalai Tesque, Inc.) to undergo blocking for ten minutes.
(2) Primary antibody reaction: the sample obtained in (1) was immersed in a diluent of an anti-annexin A2 antibody (manufactured by Cell Signaling Technology, Inc.) (at a dilution ratio of 1:200), and allowed to react for ten minutes.
(3) Washing: the sample obtained in (2) was washed.
(4) Secondary antibody reaction: the sample obtained in (3) was immersed in a diluent of an alkaline phosphatase (AP)-labeled antirabbit IgG antibody (manufactured by Jackson ImmunoResearch Inc.) (at a dilution ratio of 1:1000), and allowed to react for ten minutes.
(5) Washing: the sample obtained in (4) was washed.
(6) AP luminescence and image recording: the sample obtained in (5) was immersed in an ALP chemiluminescent substrate, Chemiluminescent AP Microwell/Membrane Substrate, Ultra Sensitive (manufactured by Surmodics, Inc.), and allowed to react for 30 minutes, followed by recording an image using a chemiluminescent photography apparatus, LumiCube (manufactured by Liponics, Inc.).
(7) Luminescence intensity measurement: the image obtained in (6) was measured for luminescence intensity using an image analysis software, Image J (NIH: National Institutes of Health).

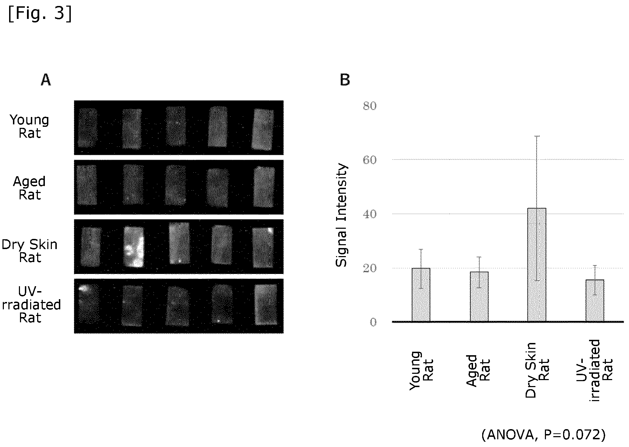

The luminescence intensity measurement results are shown in FIG. 3.
FIGs. 3A and 3B have revealed that in the dry skin rat, the skin barrier function of which was markedly decreased, Anxa 2 was detected more intensely than in the other three types of rats. In other words, they have revealed that Anxa 2 was attached to the nitrocellulose membrane in an amount that reflected variation in the skin barrier function of each rat.

## 6. Efficacy evaluation of Anxa 2 as reference marker

### (1) Sample collection

Five-week-old, nine-week-old, 13-week-old, and 25-week-old rats (SD rats, male, three rats per type) were provided. The back of each rat was shaved, and then, the shaved site was irradiated with ultraviolet light (UV-B) for ten minutes. One day after the ultraviolet irradiation, skin blotting samples were collected, immediately followed by using dissecting scissors to collect, as samples for Western blotting, skin and subcutaneous fat from the same site. Collection of skin blotting samples and collection of Western blotting samples will be below-mentioned in detail.
The skin is divided into the epidermis and dermis in this order from the surface layer. The epidermis is divided into the stratum corneum, stratum granulosum, stratum spinosum, and stratum basale, and the dermis is divided into the stratum papillare and stratum reticulare. A layer of subcutaneous adipose tissues underlies the skin, and at many sites, a layer of skeletal muscle underlies the subcutaneous adipose tissues. A skin barrier function is served mainly by the sebum barrier of the skin surface, the intercorneocyte lipid of the stratum corneum, and the tight junction of the stratum granulosum. The sample collected for Western blotting using dissecting scissors is a full-thickness skin tissue (including the epidermis, dermis, and subcutaneous adipose tissue) at the same site as the site at which the skin blotting sample was collected.
Ultraviolet light is divided into UV-A (400 to 315 nm), UV-B (315 to 280 nm), and UV-C (less than 280 nm) in this order from a longer wavelength, and the shorter the wavelength is, the stronger the chemical action is but the lower the transmittance is. The UV-B used here penetrates up to the dermal stratum papillare, and causes the increased oxidative stress, the denaturation of nucleic acids, proteins, and lipids, and the like, resulting in initiating an inflammatory reaction. Inflammatory reaction is associated with the Langerhans cell and keratinocyte existing from the stratum basale to stratum spinosum in the epidermis, the fibroblast existing in the dermis, and the inflammatory cell slipping into the dermis from the capillary vessels distributed in the dermis (neutrophil, macrophage, and the like), and these cells secrete various cytokines including TNFα, and augment or inhibit the inflammatory reaction.
Skin blotting is a method in which a nitrocellulose membrane charged with electrostatic polarity is wetted with physiological saline and applied to the skin surface to cleavage the skin barrier function temporarily, and through the clearance, a water-soluble protein in a layer located below the skin barrier is attracted and adsorbed. The attraction ranges up to not only the epidermis and dermis but also the subcutaneous fat (Non-Patent Literature 1: Minematsu et al., ADVANCES IN SKIN & WOUND CARE 2014; 27: 272-9) and the skeletal muscle (Non-Patent Literature 12: Neya et al., Abstract of the 70th Annual Meeting of the Japanese Society of Physical Fitness and Sports Medicine, and Non-Patent Literature 13: Neya et al., Abstract of the 72nd Annual Meeting of the Japanese Society of Physical Fitness and Sports Medicine). The collected nitrocellulose membrane is immunostained using an antibody, thus enabling a specific protein to be specifically detected and quantitated.

In Western blotting, a protein extracted by crushing a cell or tissue is separated by electrophoresis in accordance with the molecular weight, and then, electrically transferred to a blotting membrane such as of PVDF. The blotting membrane to which the protein was transferred was immunostained with an antibody in the same manner as in skin blotting, and thus, a specific protein is identified in accordance with the specificity of the antibody and molecular weight, enabling the luminescence intensity of the band to be used for quantitative analysis. For Western blotting, it is difficult to strictly control the amount of tissue for extracting protein and the amount of protein to be used for electrophoresis, and thus, it is common practice to simultaneously measure an internal standard protein (ACTβ, GAPDH or the like) that should have a stable expression amount in various cells, and to use the measured value to correct the luminescence intensity of a target protein.

(2) Skin blotting

(2-1) Collection of skin blotting sample

One drop of physiological saline was applied dropwise to a 1 cm × 1 cm supported nitrocellulose membrane (manufactured by Bio-Rad Laboratories, Inc.), and the resulting membrane was applied to the back skin for ten minutes, followed by detaching the membrane from the back skin. The detached nitrocellulose membrane was dried at 4°C and stored.

(2-2) Staining of skin blotting sample

The skin blotting sample collected from each rat was double-stained in accordance with the following procedures using a vacuum driven staining system, SNAP i.d. 2 (manufactured by Merck Millipore).

(a) Blocking: the collected skin blotting sample was immersed in a blocking agent, Blocking One (manufactured by Nacalai Tesque, Inc.), for ten minutes.
(b) Anxa 2 staining:

(b-1) Primary antibody reaction: the sample obtained in (a) was immersed in a diluent of an anti-Anxa 2 antibody (manufactured by Cell Signaling Technology, Inc.) (at a dilution ratio of 1:200) for ten minutes.
(b-2) Washing: the sample obtained in (b-1) was washed three times.
(b-3) Secondary antibody reaction: the sample obtained in (b-2) was immersed in a diluent of a horseradish peroxidase (HRP)-labeled antigoat IgG antibody (manufactured by Jackson ImmunoResearch Inc.) (at a dilution ratio of 1:1000) for ten minutes.
(b-4) Washing: the sample obtained in (b-3) was washed six times.
(b-5) HRP luminescence and recording: the sample obtained in (b-4) was immersed in an HRP chemiluminescent substrate, Luminata Forte (manufactured by Merck Millipore), for two minutes, and allowed to react, followed by recording an image using a chemiluminescent imager, LumiCube (manufactured by Liponics, Inc.).

(c) Quenching of HRP luminescent signal: the sample obtained in (b) was washed twice and immersed in 15% hydrogen peroxide-containing phosphate buffered saline for 30 minutes to quench the HRP luminescent signal. The sample was further washed four times and used for TNF staining.
(d) TNF-α staining:

(d-1) Primary antibody reaction: the sample obtained in (a) was immersed in a diluent of an anti-TNF-a antibody (manufactured by Cell Signaling Technology, Inc.) (at a dilution ratio of 1:200) for ten minutes.
(d-2) Washing: the sample obtained in (d-1) was washed three times.
(d-3) Secondary antibody reaction: the sample obtained in (d-2) was immersed in a diluent of an alkaline phosphatase (AP)-labeled antirabbit IgG antibody (manufactured by Jackson ImmunoResearch Inc.) (at a dilution ratio of 1:1000) for ten minutes.
(d-4) Washing: the sample obtained in (d-3) was washed six times.
(d-5) AP luminescence and recording: the sample obtained in (d-4) was immersed in an AP chemiluminescent substrate, Chemiluminescent AP Microwell/Membrane Substrate, Ultra Sensitive (manufactured by Surmodics, Inc.), for 30 minutes, and allowed to react, followed by recording an image using a chemiluminescent imager, LumiCube (manufactured by Liponics, Inc.).

(e) Luminescence intensity measurement and correction: the images obtained in (b) and (d) were measured for the luminescence intensity of Anxa 2 and TNF-α using an image analysis software, Image J (NIH). A correction was made by dividing the luminescence intensity of TNFα by the luminescence intensity of Anxa 2.

(3) Western blotting

(3-1) Collection of Western blotting sample

Immediately after the skin blotting sample was collected, a full-thickness skin tissue (including the epidermis, dermis, and subcutaneous adipose tissue) at the same site was collected using dissecting scissors. The collected tissue was immersed in a RIPA buffer (manufactured by Nacalai Tesque, Inc.) to extract protein, to which sodium lauryl sulfate (SDS) was added, and the resulting mixture was boiled.

(3-2) Electrophoresis and blotting

A 10% acrylamide gel was produced, and each sample was separated by electrophoresis (200 V, 40 minutes). The separated protein was transferred to a PVDF membrane using a dry blotting system, iBlot (manufactured by Invitrogen), (Western blotting sample). Two Western blotting samples were produced.

(3-3) Staining of PVDF membrane

Using a vacuum driven staining system, SNAP i.d. 2 (manufactured by Merck Millipore), one of the two Western blotting samples was used for TNFα staining, and the other sample was used for actin β (ACTβ) staining. The staining procedures are as below-mentioned.

(a) Blocking: the Western blotting sample was immersed in a blocking agent, Blocking One (manufactured by Nacalai Tesque, Inc.), for ten minutes.
(b) Primary antibody reaction: the sample obtained in (a) was immersed in a diluent of an anti-ACTβ antibody (manufactured by Cell Signaling Technology, Inc.) (at a dilution ratio of 1:1000) or a diluent of an anti-TNF-a antibody (manufactured by Cell Signaling Technology, Inc.) (at a dilution ratio of 1:200) for ten minutes.
(c) Washing: the sample obtained in (b) was washed three times.
(d) Secondary antibody reaction: the sample obtained in (c) was immersed in a diluent of a horseradish peroxidase (HRP)-labeled antirabbit IgG antibody (manufactured by Jackson ImmunoResearch Inc.) (at a dilution ratio of 1:1000) for ten minutes.
(e) Washing: the sample obtained in (d) was washed six times.
(f) HRP luminescence and recording: the sample obtained in
(e) was immersed in an HRP chemiluminescent substrate, Luminata Forte (manufactured by Merck Millipore), for two minutes, and allowed to react, followed by recording an image using a chemiluminescent imager, LumiCube (manufactured by Liponics, Inc.).

(3-4) Luminescence intensity measurement and correction:
The TNFα staining image and ACTβ staining image obtained in (f) were measured for the luminescence intensity of the respective specific bands using an image analysis software, Image J (produced by NIH), and then, a correction was made by dividing the luminescence intensity of TNFα by the luminescence intensity of ACTβ.

The corrected luminescence intensity of TNFα in Western blotting [WB (TNFα/ACTβ)] and the uncorrected luminescence intensity [SB (TNFα)] and corrected luminescence intensity [SB (TNFα/Anxa2)] of TNFα in skin blotting are shown in Table 5.

[Table 5]

| Animal ID | WB (TNFα/ACTβ) | SB (TNFα) | SB (TNFα/ANXA2) |
|---|---|---|---|
| 5wk-1 | 0.77 | 17.40 | 0.75 |
| 5wk-2 | 0.55 | 39.33 | 1.07 |
| 5wk-3 | 0.52 | 16.33 | 1.14 |
| 9wk-1 | 1.19 | 21.72 | 3.78 |
| 9wk-2 | 0.82 | 35.37 | 4.56 |
| 9wk-3 | 1.06 | 12.93 | 1.75 |

(continued)

| Animal ID | WB (TNFα/ACTβ) | SB (TNFα) | SB (TNFα/ANXA2) |
|---|---|---|---|
| 13wk-1 | 1.23 | 14.27 | 3.16 |
| 13wk-2 | 1.20 | 20.42 | 4.87 |
| 13wk-3 | 0.55 | 9.45 | 2.78 |
| 25wk-1 | 1.67 | 27.67 | 3.99 |
| 25wk-2 | 1.29 | 12.74 | 3.41 |
| 25wk-3 | 0.74 | 6.74 | 0.99 |
| r | | 0.02 | 0.63 |
| p | | 0.96 | 0.03 |

The corrected luminescence intensity in Western blotting reflects the amount of TNFα expressed in the stratum spinosum and stratum basale of the epidermis and in the dermal stratum papillare by the inflammatory reaction due to ultraviolet irradiation. The correlation between the uncorrected/corrected luminescence intensities of TNFα detected in skin blotting and the corrected luminescence intensity of TNFα quantitated by Western blotting was subjected to Pearson's correlation analysis, with the result that the uncorrected luminescence intensity exhibited no correlation (correlation coefficient r = 0.02) but that the corrected luminescence intensity exhibited a significant correlation (correlation coefficient r = 0.63). This indicates that Anxa 2 is useful as a reference marker in skin blotting.

**Claims**

1. A method of assisting diagnosis of a disease in a subject using a disease marker and a reference marker, the method comprising the following steps of:

   (a) providing a sheet capable of generating an attractive force due to electrostatic interaction between the sheet and each of the disease and reference markers;
   (b) applying the sheet to a skin surface of the subject, followed by detaching the sheet from the skin surface;
   (c) measuring an amount of each of the disease and reference markers that are attached to the sheet; and
   (d) acquiring information on the disease in the subject by using a value obtained by correcting the amount of the disease marker measured in step (c) with the amount of the reference marker measured in step (c);

   wherein the reference marker is annexin A2.

2. A method of collecting a disease marker and a reference marker from a subject to assist diagnosis of a disease in the subject using the disease marker and the reference marker,
the method comprising the following steps of:

   (a) providing a sheet capable of generating an attractive force due to electrostatic interaction between the sheet and each of the disease and reference markers; and
   (b) applying the sheet to a skin surface of the subject, followed by detaching the sheet from the skin surface;

   wherein the reference marker is annexin A2.

3. The method according to claim 1 or 2, wherein the disease marker is a protein selected from the group consisting of cytokines, enzymes, extracellular matrix proteins, plasma proteins, heat-shock proteins, and receptor proteins, or is an osmolyte.

4. The method according to any one of claims 1 to 3, wherein the disease marker is a protein selected from the group consisting of TNFα, IL1α, NGFβ, VEGF-C, TGFβ1, BMP1, PAI1, MMP2, creatine kinase, creatine phosphokinase, COL4, fibronectin, albumin, HSP90α, and NOTCH1, or is taurine.

5. The method according to any one of claims 1 to 4, wherein the sheet has a polar functional group on a surface thereof.

6. The method according to claim 5, wherein the polar functional group is a nitro group.

7. The method according to claim 6, wherein the sheet is a nitrocellulose membrane.

8. The method according to claim 7, wherein the nitrocellulose membrane has a nitration ratio of 30% or more.

9. The method according to claim 5, wherein the polar functional group is a cationic functional group.

10. The method according to claim 5, wherein the polar functional group is an anionic functional group.

11. The method according to any one of claims 1 to 10, wherein, in step (b), one or both of the sheet and the skin surface is/are wetted with an aqueous medium, and then, the sheet is applied to the skin surface.

12. A kit for assisting diagnosis of a disease in a subject using a disease marker and a reference marker,
the kit comprising
a sheet capable of generating an attractive force due to electrostatic interaction between the sheet and each of the disease and reference markers,
wherein the reference marker is annexin A2.

13. A kit for collecting a disease marker and a reference marker from a subject to assist diagnosis of a disease in the subject using the disease marker and the reference marker,
the kit comprising
a sheet capable of generating an attractive force due to electrostatic interaction between the sheet and each of the disease and reference markers,
wherein the reference marker is annexin A2.

14. The kit according to claim 12 or 13, wherein the disease marker is a protein selected from the group consisting of cytokines, enzymes, extracellular matrix proteins, plasma proteins, heat-shock proteins, and receptor proteins, or is an osmolyte.

15. The kit according to any one of claims 12 to 14, wherein the disease marker is a protein selected from the group consisting of TNFα, IL1α, NGFβ, VEGF-C, TGFβ1, BMP1, PAI1, MMP2, creatine kinase, creatine phosphokinase, COL4, fibronectin, albumin, HSP90α, and NOTCH1, or is taurine.

16. The kit according to any one of claims 12 to 15, wherein the sheet has a polar functional group on a surface thereof.

17. The kit according to claim 16, wherein the polar functional group is a nitro group.

**18.** The kit according to claim 17, wherein the sheet is a nitrocellulose membrane.

**19.** The kit according to claim 18, wherein the nitrocellulose membrane has a nitration ratio of 30% or more.

**20.** The kit according to claim 16, wherein the polar functional group is a cationic functional group.

**21.** The kit according to claim 16, wherein the polar functional group is an anionic functional group.

**22.** The kit according to any one of claims 12 to 21, further comprising a first reagent for detecting the disease marker and a second reagent for detecting the reference marker.

[Fig. 1]

*P<0.05 in comparison with young rat

[Fig. 2]

[Fig. 3]

A

Young Rat

Aged Rat

Dry Skin Rat

UV-rradiated Rat

B

Signal Intensity

80

60

40

20

0

Young Rat

Aged Rat

Dry Skin Rat

UV-irradiated Rat

(ANOVA, P=0.072)

## INTERNATIONAL SEARCH REPORT

| International application No. |
| --- |
| PCT/JP2019/014667 |

**A. CLASSIFICATION OF SUBJECT MATTER**
Int.Cl. G01N33/68(2006.01)i, G01N1/04(2006.01)i, G01N33/50(2006.01)i

According to International Patent Classification (IPC) or to both national classification and IPC

**B. FIELDS SEARCHED**

Minimum documentation searched (classification system followed by classification symbols)
Int.Cl. G01N33/68, G01N1/04, G01N33/50

| Documentation searched other than minimum documentation to the extent that such documents are included in the fields searched | |
| --- | --- |
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)
JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
| --- | --- | --- |
| A | JP 2018-48984 A (THE UNIVERSITY OF TOKYO) 29 March 2018, claims & WO 2018/056418 A1 | 1-22 |
| A | JP 2017-198509 A (KINDAI UNIVERSITY) 02 November 2017, paragraph [0083] (Family: none) | 1-22 |
| A | JP 2015-227865 A (KAO CORP.) 17 December 2015, paragraph [0003] & US 2017/0192015 A1, paragraph [0003] & WO 2015/170781 A1 & EP 3036340 A1 & CN 106460031 A | 1-22 |

☐ Further documents are listed in the continuation of Box C.    ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 28 June 2019 (28.06.2019) | 09 July 2019 (09.07.2019) |

| Name and mailing address of the ISA/ | Authorized officer |
| --- | --- |
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**REFERENCES CITED IN THE DESCRIPTION**

**Patent documents cited in the description**

- JP 2018048984 A


**Non-patent literature cited in the description**

- **MINEMATSU et al.** *ADVANCES IN SKIN & WOUND CARE,* 2014, vol. 27, 272-9
- **OGAI et al.** *International Journal of Cosmetic Science,* 2015, vol. 27, 425-432
- **OGAI et al.** *International Journal of Cosmetic Science,* 2016, vol. 38, 462-469
- **KISHI et al.** *Biological Research for Nursing,* 2015, vol. 17 (2), 135-141
- **KOYANO et al.** *International Wound Journal,* 2016, vol. 13 (2), 189-97
- **TAMAI et al.** *Journal of Nursing Science and Engineering,* 2017, vol. 4 (2), 116-120
- **KANEKO et al.** *Wound Repair and Regeneration,* 2015, vol. 23, 657-663
- **KANAZAWA et al.** *PLOS ONE,* 2014, vol. 9 (8), 1-11
- **KIMURA.** Master's Thesis. Division of Health Sciences and Nursing, Graduate School of Medicine, The University of Tokyo, 2016
- **KIMURA et al.** *Abstract of Oral Presentation at the 46th Annual Meeting of the Japanese Society for Wound Healing*
- **NAKAI.** Master's Thesis. Division of Health Sciences and Nursing, Graduate School of Medicine, The University of Tokyo, 2018
- **NEYA et al.** *Abstract of the 70th AnnualMeeting of the Japanese Society of Physical Fitness and Sports Medicine*
- **NEYA et al.** *Abstract of the 72nd Annual Meeting of the Japanese Society of Physical Fitness and Sports Medicine*
- **SARI et al.** *WOUNDS,* 2010, vol. 22 (2), 44-51
- **MINEMATSU et al.** *Abstract of the 19th Annual Meeting of Japan Society of Clinical Hair Restoration*
- **MUGITA et al.** *PLOS One,* 2015, vol. 10, e0138117
- **MUGITA et al.** *Int Wound J.,* 2018, vol. 15, 623-32
- **ZHU et al.** *Sci Rep,* 2011, vol. 1, 23
- **SZEGEDI et al.** *J Eur Acad Dermatol Venereol.,* 2015, vol. 29, 2136-44
- **TAN et al.** *J Cutan Med Surg.,* 2017, vol. 21, 308-315

- **BRUNNER et al.** *J Allergy Clin Immunol,* 2019, vol. 143, 142-154
- **TRZNADEL-GRODZKA et al.** *Postepy Hig Med Dosw,* 2012, vol. 66, 843-847
- **WIKRAMANAYAKE et al.** *Exp Dermatol.,* 2018, vol. 27, 1408-1411
- **TOKURA et al.** *J Am Acad Dermatol,* 2007, vol. 57, S22-S25
- **HASHIMOTO et al.** *J Dermatol,* 2007, vol. 34, 577-582
- **HEROUY et al.** *J Dermatol Sci,* 2001, vol. 25, 198-205
- **DIANIS et al.** *9th World Congress on Itch. Wroclaw (Poland),* 15 October 2017
- **USHIGOME et al.** *Clin Exp Allergy,* 2018, vol. 48, 1453-1463
- **UETA et al.** *BMJ Open Ophthalmol,* 2017, vol. 1, e000073
- **WANG et al.** *J Clin Invest,* 2018, vol. 128, 985-996
- **BREMBILLA et al.** *Front Immunol.,* 2018, vol. 9, 1682
- **EBRAHIM et al.** *Int J Trichology,* 2019, vol. 11, 26-30
- **GONG et al.** *Exp Dermatol,* 2018
- **MORAVVEJ et al.** *Hum Antibodies,* 2018, vol. 26, 201-207
- **CELIK ; ATES.** *J Clin Lab Anal.,* 2018, vol. 32, e22386
- **MINEMATSU et al.** *Abstract of the 20th Annual Meeting of Japan Society of Clinical Hair Restoration*
- **MANSOURI et al.** *Br J Dermatol,* 2016, vol. 174, 916-918
- **WERNINGHAUS et al.** *Clin Exp Dermatol,* 2018, vol. 43, 458-459
- **TAKEHARA et al.** *the 3rd Annual Meeting of The Society of Nursing Science and Engineering (Abstract)*
- **TAKEHARA et al.** *the 46th Annual Meeting of the Japanese Society for Wound Healing (Abstract)*
- **PELLEGRINI et al.** *PLOS One,* 2017, vol. 12, e0183415

- **WU et al.** *Cytokine,* 2018, vol. 110, 94-103
- **HAIMAKAINEN et al.** *Cancer Invest,* 2017, vol. 35, 143-151
- **ZHU et al.** *Gastroenterology Res,* 2017, vol. 10, 65-69
- **MINEMATSU et al.** *Japanese Journal of Pressure Ulcers,* 2014, vol. 16, 154-158
- **BRIDGE et al.** *Front Med,* 2018, vol. 5, 351
- **KOYANO et al.** *Int Wound J.,* 2016, vol. 13, 189-197
- **NAKAI et al.** *Journal of Tissue Viability,* 2019
- **KIMURA et al.** *the 46th Annual Meeting of the Japanese Society for Wound Healing (Abstract)*
- **NEYA et al.** *ASCA International Conference on Applied Strength & Conditioning (Abstract),* 2018
- **NEYA et al.** *the 72nd AnnualMeeting of the Japanese Society of Physical Fitness and Sports Medicine (Abstract)*
- **NEYA et al.** *the 70th Annual Meeting of the Japanese Society of Physical Fitness and Sports Medicine (Abstract),* 16 September 2017
- **SAARINEN ; SIIMES.** *Acta Paediatr Scand,* 1978, vol. 67, 745-751
- **SHENKIN.** *Clin Chem,* 2006, vol. 52, 2177-2179
- **GAMA-AXELSSON.** *Clin J Am Soc Nephrol.,* 2012, vol. 7, 1446-1453
- **HIGASHIMURA et al.** *Joint Meeting of the 5th Annual Meeting of The Society for Nursing Science and Engineering, the 11th Annual Meeting of the Society of Nursing Practice, and the 7th Annual Meeting of International Lymphoedema Framework Japan (Abstract)*
- **HIGASHIMURA et al.** *the 23rd Annual Meeting of Japan Academy of Gerontological Nursing (Abstract)*
- **MINEMATSU et al.** *the 68th Meeting of The Japan Geriatrics Society - the Kanto Koshinetsu Regional Meeting (Abstract)*
- *Analytica Chimica Acta,* 2011, vol. 685, 196-203
- **EISENBERG E ; LEVANON EY.** *Trends Genet.,* 2003, vol. 19 (7), 362-5
- Luminescence intensity measurement: the image obtained in (6) was measured for luminescence intensity using an image analysis software. Image J. NIH: National Institutes of Health
- **NEYA et al.** *Abstract of the 70th Annual Meeting of the Japanese Society of Physical Fitness and Sports Medicine*
- Luminescence intensity measurement and correction: the images obtained in (b) and (d) were measured for the luminescence intensity of Anxa 2 and TNF-$\alpha$ using an image analysis software. Image J. NIH